# EUROPEAN PATENT SPECIFICATION

(11) **EP 2 035 828 B1**
(45) Date of publication and mention of the grant of the patent: **28.11.2012**
(21) Application number: 07725744.2
(22) Date of filing: 01.06.2007
(51) Int. Cl.: G01N 33/50, G01N 33/68, C12N 15/79, C12N 15/81

(54) **METHODS AND TOOLS FOR THE SCREENING OF FACTORS AFFECTING PROTEIN MISFOLDING**
VERFAHREN UND WERKZEUGE ZUM SCREENING VON DIE FEHLFALTUNG VON PROTEINEN BEEINFLUSSENDEN FAKTOREN
PROCÉDÉS ET OUTILS POUR LE CRIBLAGE DE FACTEURS MODIFIANT LE MAUVAIS REPLIEMENT DE PROTÉINES

(30) Priority: 02.06.2006 GB 0610792
(43) Date of publication of application: 18.03.2009
(73) Proprietor: NV reMYND, 3001 Leuven-Heverlee (BE)
(72) Inventor: GRIFFIOEN, Gerard, B-3210 Linden (BE); COUPET, Kristel, Marie, Edith, B-1700 Dilbeek (BE); VAN DAMME, Nele, B-3010 Kessel-Lo (BE); WERA, Stefaan, B-3360 Bierbeek (BE)
(74) Representative: Bird, William Edward
(86) International application number: PCT/EP2007/004864
(87) International publication number: WO 2007/140933

(56) References cited:
- EP-A- 1 793 001
- WO-A-03/004698
- WO-A-2004/090532
- WO-A-2004/093790
- K.A. BRANDIS ET AL: "alpha-Synuclein fission yeast model: concentration-dependent aggregation without plasma membrane localization or toxicity." JOURNAL OF MOLECULAR NEUROSCIENCE : MN 2006, vol. 28, no. 2, 1 February 2006 (2006-02-01), pages 179-191, XP008083781 Totowa NJ USA ISSN: 0895-8696

### SEQUENCE LISTING

<110> nv ReMynd
<120> Methods and tools for the screening of factors affecting protein misfolding
<130> R4288-PCT
<150> GB0610792.4
   <151> 2006-06-02
<160> 4
<170> PatentIn version 3.3
<210> 1
   <211> 100
   <212> DNA
   <213> Artificial
<220>
   <223> oligonucleotide
<400> 1
   cttgtatgtc aatgtttgtc actaaacgga tagaataggt actaaacgct acaaagaaaa    60
   atgacagagc agaaagccct agtaaagcgt attacaaatg                          100
<210> 2
   <211> 94
   <212> DNA
   <213> Artificial
<220>
   <223> oligonucleotide
<400> 2
   cgccatcgta taaaaggtta aacataaaaa gtagctaaat agaacactat agaagaataa     60
   ctacataaga acacctttgg tggagggaac atcg                                 94
<210> 3
   <211> 46
   <212> DNA
   <213> Artificial
<220>
   <223> oligonucleotide
<400> 3
   ggaattcgga gctctatata tatttcaagg atataccatt ctaatg 46
<210> 4
   <211> 43
   <212> DNA
   <213> Artificial
<220>
   <223> oligonucleotide
<400> 4
   ggaattcgcc tgcaggcatc tccatgcagt tggacgatcg atg 43

## Description

### FIELD OF THE INVENTION

The present invention relates in general to the fields of chemical, pharmaceutical and genetic screening. In particular, it discloses engineered yeast cells and systems based thereon that can be used to screen for substances or conditions that affect protein misfolding.

### BACKGROUND OF THE INVENTION

Proper folding of polypeptides into three-dimensional structures is an essential feature to form biologically active proteins and therefore pivotal for cellular functioning. When compromised, however, improper folding intermediates arise that often are unstable and non-functional and even may possess cytotoxic properties such as the propensity to self-associate, eventually leading to formation of insoluble deposits. Proteins adopting an altered secondary, tertiary and/or quaternary structure can lead to a functional deficit of the natively folded, biologically active protein, as observed e.g. in cystic fibrosis or Marfan syndrome. Alternatively or concomitantly, the abnormally folded form of the protein is associated with a toxic gain of function, as seen in several neurodegenerative diseases.

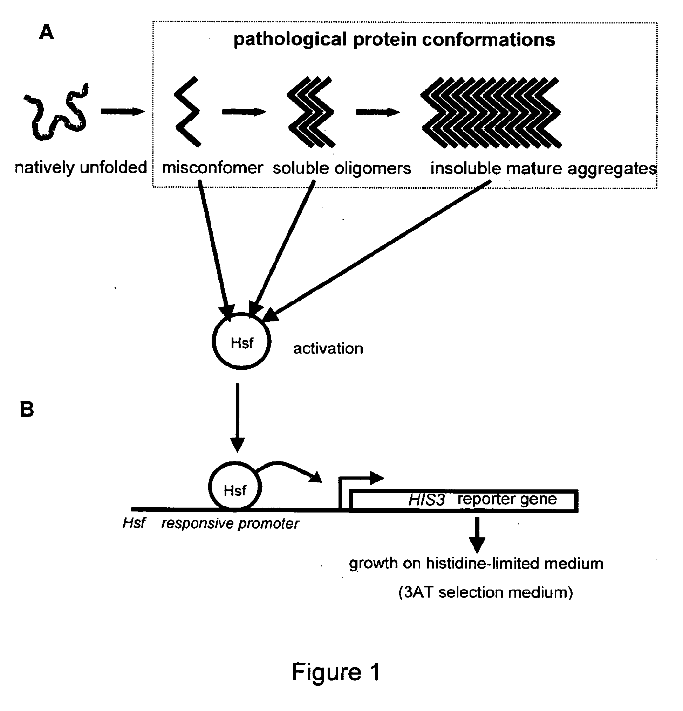

A host of human diseases are associated with aberrant protein folding (and subsequent aggregation), such as cystic fibrosis, the serpinopathies, prion diseases, and most notably, several neurodegenerative diseases, such as Alzheimer's, Parkinson's and Huntington's disease. These latter three disorders are characterised by noxious polymerisation of specific proteins (amyloid precursor protein, tau, α-synuclein, huntingtin) into insoluble aggregates which has been suggested to eventually culminate into cellular degeneration and cell death (Fig. 1, upper part). Compelling evidence for such a scenario comes from identification of dominant familial mutations in genes encoding amyloidogenic proteins (e.g. amyloid precursor protein, tau, α-synuclein) which cause early disease onset in individuals bearing such alleles. The insoluble aggregates are often referred to as amyloid or amyloid-like deposits (Latin 'amylum' = starch) and show a characteristic β-sheet structure and have distinct tinctorial properties (e.g. displaying red-green or apple-green birefringence under polarised light following staining with Congo red).

Although the presence of amyloid-like deposits constitutes a very distinct pathological hallmark there is a growing body of evidence that in fact soluble monomeric or oligomeric precursors of fibril formation (protofibrils) are the principal culprits triggering cellular degeneration (Bucciantini et al., Nature. 2002 416(6880): 507-11). In fact some studies suggest that the insoluble aggregates are biologically inert and may even constitute cytoprotective reservoirs of otherwise toxic oligomeric precursors (Walsh et al., Nature. 2002 416(6880): 535-9; Caughey and Lansbury, Annu Rev Neurosci. 2003 (26): 267-298). In line with this observation, therapeutic intervention should be focussed on preventing formation of toxic misconformers (or to neutralise their noxious effects on cellular integrity) rather than just to dissolve existing (or to prevent the formation of) inert aggregates.

Importantly, other protein misfolding diseases, e.g. the serpinopathies, are also characterized by harmful accumulation and deposition of proteins in insoluble aggregates. Although these deposits do not display the typical cross-β structure of amyloids, the disease mechanism is very similar to amyloid deposition diseases: an aberrantly folded form of the protein starts to aggregate into oligomers and further accumulation leads to sequestering of the protein in insoluble aggregates. This disease mechanism might be more widespread than originally anticipated: the misfolding of cystic fibrosis transmembrane conductance regulator (CFTR) is generally accepted to be the cause of cystic fibrosis. Recently, it was shown that misfolded CFTR is able to aggregate and form large microtubule-dependent cytoplasmic inclusion bodies called aggresomes, although it is not proven that aggregation contributes to pathogenesis.

The proteins implicated in these 'protein misfolding diseases' display no apparent structural or sequence homology, but the mechanism underlying these diverse disorders is believed to be the same, and the resulting diseases are referred to as conformational diseases. Although the precise mechanism of protein aggregation is at present elusive, it involves formation of misfolded (or partially denatured) monomeric intermediates possessing intrinsic properties to self-polymerise in stable *de facto* alternative (oligomeric) conformations (Fig. 1, upper part). Since conformational diseases are characterised by aberrant folding and then aggregation of the underlying protein, the hallmark of such protein is its inherent ability to adopt at least two different stable conformations.

In healthy cells surveillance systems actively prevent deleterious accumulation of misfolded proteins either by refolding them in native form or by eliminating them altogether. One such 'quality control' constitutes activation of transcriptional activator Hsf1 (heat shock factor 1) by denaturated peptides. Hsf1 activation leads to increased synthesis of protein chaperones that facilitate re-folding into native states or alternatively may direct to degradation. Not surprisingly, when such surveillance systems are compromised (for instance in aged cells) the clearance of aberrant proteins may not be sufficient to prevent toxic aggregation of proteins, as seen in e.g. amyloidogenesis.

In view of the pathological role of aberrant folding of proteins in various diseases, there is a clear need for efficient screening methods which allow the identification of compounds that can either directly or indirectly affect protein misfolding and/or aggregation.

Brandis et al. (2006) J. Mol. Neurosci. 28, 179-191, describe yeast cells wherein wild type and mutant alpha-synuclein are placed under the control of a thiamine repressible promoter and are used to evaluate alpha synuclein misfolding, aggregation and toxicity.

PCT patent application W003004698 describes methods for detecting protein (mis)folding wherein a reporter gene is used which is under the control of the promoter of the periplasmatic protease DegP. In bacteria, the presence of misfolded proteins leads to an induction of this protease.

PCT patent application W004093790 describes yeast cells which express alpha synuclein under the control of an inducible promoter (typically nutrient or hormone responsive promoters).

PCT patent application W004090532 relates to the monitoring of xenografts using reporter constructs which detect e.g. protein stability of proteins in such a graft.

EP1799001 describes yeast cells comprising alpha synuclein and a reporter construct. The application describes various types of promoters but is silent on specific promoters for the reporter construct.

Cho et al. (2002), Abstr Intersci Conf Antimicrob Agents Chemother 2002, Sept 27-30, 42, abstract no. F-754, discloses an engineered Mycobacterium bacterial cell for testing the viability of these bacteria after antibiotic treatment. The assay is based on a red fluorescent protein reporter system under the control of a heat shock promoter.

### SUMMARY OF THE INVENTION

The invention as claimed relates in a first aspect to methods for determining whether a compound or a plurality of compounds is/are capable of directly or indirectly affecting protein misfolding. These methods comprise the steps of:
a) providing a culture of engineered yeast cells, comprising one or more reporter gene(s) under transcriptional control of a promoter of a heat shock protein, and further comprising one or more gene(s) encoding one or more protein(s) prone to misfolding, wherein the engineered yeast cells are characterized by spontaneous or inducible misfolding of the protein(s) prone to misfolding; and
b) contacting the cells with the compound or the plurality of compounds and detecting the activity of the reporter gene product in the cells to determine the effect of the compound or the plurality of compounds on protein misfolding in the engineered cells.
Wherein the protein prone to misfolding is selected from the group consisting of serpins, poly(A) binding protein 2, low-density lipoprotein receptor, apolipoprotein B100, cochlin, RET, myelin protein 22/0, short-chain acyl CoA dehydrogenase, cystic fibrosis transmembrane conductance regulator, HERG, phenylalanine hydroxylase, fibrillin, procollagen, collagen, haemoglobin, ATPase ATP7B, β-hexosaminidase tyrosinase, amyloid precursor protein, tau, α-synuclein, huntingtin, ADan peptide, ABri peptide, amyloid β, prion protein, ataxins, superoxide dismutase, cystatin C, atrophin 1, androgen receptor, TATA box-binding protein, transthyretin, serum amyloid A, Ig light chains, β2-microglobulin, apolipoprotein A-1, gelsolin, pro- islet amyloid polypeptide, procalcitonin, lysozyme, insulin, fibrinogen α-chain, atrial natriuretic factor, surfactant protein C, rhodopsin, γ-crystallins, lactoferrin, βig-h3, kerato-epithelin, corneodesmosin, p53, lactadherin and prolactin.

Embodiments of these methods are methods, which are methods for screening a plurality of compounds capable of directly or indirectly affecting protein misfolding, wherein the compounds are cDNAs or the expression products thereof, comprising the steps of:
a) providing a culture of engineered yeast cells comprising one or more reporter gene(s) under transcriptional control of a promoter of a heat shock protein, and one or more gene(s) encoding one or more protein(s) prone to misfolding, the engineered yeast cell being characterized by spontaneous or inducible misfolding of the protein(s) prone to misfolding;
b) transforming the culture of engineered cells with an expression cDNA library so as to obtain a culture of transformed cells each comprising one or more cDNAs; and,
c) detecting the activity of the reporter gene in the transformed cells of the culture to determine the effect of the expression product of the one or more cDNAs on misfolding in the transformed cells.

In certain embodiments, the engineered yeast cells are characterized by spontaneous misfolding of the one or more protein(s) prone to misfolding and step (a) comprises:
- cultivating engineered yeast cells comprising one or more reporter gene(s) under transcriptional control of a promoter of a heat shock protein and one or more gene(s) encoding one or more protein(s) prone to misfolding, under conditions which allow and/or induce mutations of the genome of the engineered yeast cells resulting in spontaneous misfolding of the protein(s) prone to misfolding, and
- selecting a cell characterized by spontaneous misfolding based on increased expression of the one or more reporter gene(s) compared to the engineered yeast cells before cultivation.

In certain embodiments of these methods the cells are characterized by inducible misfolding of the protein(s) prone to misfolding and the method further comprises, prior to step (b) the step of contacting the engineered yeast cells with one or more external factors capable of selectively inducing misfolding of the one or more proteins prone to misfolding.

In particular embodiments, at least one of the protein(s) prone to misfolding is α-synuclein and the external factor capable of selectively inducing misfolding of the one or more protein(s) prone to misfolding is selected from the group consisting of paraquat, rotenone and MPTP.

In particular embodiments, at least one of the one or more protein(s) prone to protein misfolding is an amyloidogenic protein selected from the group consisting of amyloid precursor protein, tau, α-synuclein, huntingtin, ADan peptide, ABri peptide, amyloid β, prion protein, ataxins, superoxide dismutase, cystatin C, atrophin 1, androgen receptor, TATA box-binding protein, transthyretin, serum amyloid A, Ig light chains, β2-microglobulin, apolipoprotein A-1, gelsolin, pro- islet amyloid polypeptide, procalcitonin, lysozyme, insulin, fibrinogen α-chain, atrial natriuretic factor, surfactant protein C, rhodopsin, γ-crystallins, lactoferrin, βig-h3, kerato-epithelin, corneodesmosin, p53, lactadherin and prolactin.

In particular embodiments, at least one of the one or more reporter genes encodes a protein with an activity that positively or negatively affects growth and/or proliferation of the engineered yeast cells.

The invention as claimed relates in a second aspect to yeast cells, having a transgene present as an extrachromosomal element or integrated into its genomic DNA, the cell comprising:
a) one or more reporter gene(s) under transcriptional control of a promoter of a heat shock protein; and
b) one or more transgene(s) encoding one or more protein(s) prone to misfolding, wherein the protein prone to misfolding is selected from the group consisting of serpins, poly(A) binding protein 2, low-density lipoprotein receptor, apolipoprotein B100, cochlin, RET, myelin protein 22/0, short-chain acyl CoA dehydrogenase, cystic fibrosis transmembrane conductance regulator, HERG, phenylalanine hydroxylase, fibrillin, procollagen, collagen, haemoglobin, ATPase ATP7B, β-hexosaminidase tyrosinase, amyloid precursor protein, tau, α-synuclein, huntingtin, ADan peptide, ABri peptide, amyloid β, prion protein, ataxins, superoxide dismutase, cystatin C, atrophin 1, androgen receptor, TATA box-binding protein, transthyretin, serum amyloid A, Ig light chains, β2-microglobulin, apolipoprotein A-1, gelsolin, pro- islet amyloid polypeptide, procalcitonin, lysozyme, insulin, fibrinogen α-chain, atrial natriuretic factor, surfactant protein C, rhodopsin, γ-crystallins, lactoferrin, βig-h3, kerato-epithelin, corneodesmosin, p53, lactadherin and prolactin

The invention as claimed further relates to a culture of yeast cells as described under the second aspect, characterized in that the cells comprise an expression vector comprising a cDNA.

The invention as claimed relates to methods to generate a yeast cell for monitoring protein misfolding, comprising the steps of:
a) providing engineered yeast cells comprising:
   - one or more reporter gene(s) under transcriptional control of a promoter of a heat shock protein, and
   - one or more genes encoding one or more proteins prone to misfolding,
b) cultivating the yeast cells under conditions which allow mutations of the genome of the yeast cells; and
c) selecting a cell wherein the one or more reporter gene(s) are expressed at higher levels, compared to the yeast cells prior to the cultivation
wherein the protein prone to misfolding is selected from the group consisting of serpins, poly(A) binding protein 2, low-density lipoprotein receptor, apolipoprotein B100, cochlin, RET, myelin protein 22/0, short-chain acyl CoA dehydrogenase, cystic fibrosis transmembrane conductance regulator, HERG, phenylalanine hydroxylase, fibrillin, procollagen, collagen, haemoglobin, ATPase ATP7B, β-hexosaminidase tyrosinase, amyloid precursor protein, tau, α-synuclein, huntingtin, ADan peptide, ABri peptide, amyloid β, prion protein, ataxins, superoxide dismutase, cystatin C, atrophin 1, androgen receptor, TATA box-binding protein, transthyretin, serum amyloid A, Ig light chains, β2-microglobulin, apolipoprotein A-1, gelsolin, pro-islet amyloid polypeptide, procalcitonin, lysozyme, insulin, fibrinogen α-chain, atrial natriuretic factor, surfactant protein C, rhodopsin, γ-crystallins, lactoferrin, βig-h3, kerato-epithelin, corneodesmosin, p53, lactadherin and prolactin

In particular embodiments thereof, the cells are provided with a gene encoding a counterselection marker linked to the one or more genes encoding one or more proteins prone to misfolding and the method comprises the step of evaluating, by use of the counterselection marker, whether the reporter gene expression levels are dependent on the presence of the one or more genes encoding one or more proteins prone to misfolding.

The invention as claimed further relate to the use of a cell according to the second aspect, for the identification of compounds and/or conditions affecting protein misfolding, by detecting the activity of the reporter gene product in the cells upon application of the compounds and/or conditions.

Quite a number of diseases are associated with an aberration in the protein folding process. This invention provides methods to monitor protein misfolding in living cells and therefore constitutes an important tool to develop therapeutics for treatment of diseases that involve (aberrant) protein conformations.

To this end, the invention provides engineered yeast cells comprising one or more reporter gene(s) under transcriptional control of a promoter of a heat shock protein and one or more gene(s) encoding one or more protein(s) prone to misfolding. In a particular aspect of the invention, at least one of the one or more protein(s) prone to misfolding is encoded by a transgene. According to a particular embodiment at least one of the protein(s) prone to protein misfolding is an amyloidogenic protein. In one embodiment, at least one of the protein(s) prone to misfolding is a human protein. More particularly, the engineered cells of the present invention comprise a gene encoding human tau or human α-synuclein.

According to one embodiment the one or more of the one or more genes encoding the one or more proteins prone to protein misfolding present in the engineered cell of the invention are minigenes.

The cells provided for use in the methods and assays of the present invention are cells in which misfolding of the one or more proteins prone to misfolding either occurs spontaneously or can be induced by external factors. According to a particular embodiment of the invention, misfolding of the protein prone to misfolding occurs spontaneously in the cell. Such a cell characterized by spontaneous misfolding can be obtained by cultivating engineered yeast cells comprising one or more reporter gene(s) under transcriptional control of a promoter of a heat shock protein and one or more gene(s) encoding one or more protein(s) prone to misfolding under conditions which allow or induce mutations in the cells and selecting a cell line or strain wherein the expression of the one or more reporter gene(s) is increased compared to the expression of the one or more reporter gene(s) prior to cultivation and selection.

The present invention relates to engineered yeast cells which are characterized by spontaneous protein misfolding in which spontaneous protein misfolding occurs as a result of spontaneous mutations occurring during cultivation, which cells have been selected based on increased expression of the reporter gene.

In an alternative embodiment, the yeast cells of the invention are cells in which misfolding of the one or more proteins prone to misfolding can be induced by contacting the cell with external factors. According to a particular embodiment the cells of the invention comprise a protein prone to misfolding which is alpha-synuclein and the external factor is a factor such as, but not limited to paraquat, rotenone or MPTP (1-methyl 4-phenyl 1,2,3,6-tetrahydropyridine).

The promoters of the one or more reporter genes present in engineered cells according to particular embodiments of the present invention are characterized in that they are responsive to protein misfolding, i.e. expression of the reporter gene is activated by the occurrence of protein misfolding in the cell. More particularly, the promoter of a heat shock protein is a promoter activated by Hsf1. Most particularly, the promoter is the yeast *SSA3* promoter.

The nature of the reporter gene activity is not critical. According to one embodiment at least one of the one or more reporter genes encodes a protein with an activity that positively or negatively affects growth and/or proliferation of the engineered yeast cells. Most particularly at least one of the one or more reporter genes present in the engineered yeast cells of the invention encodes a protein encoding a compound required for the synthesis of an essential amino acid. In such cells, expression of the reporter gene can be determined by cultivating the cells in a medium not containing the essential amino acid. Additionally or alternatively, at least one of the one or more reporter genes present in the engineered cells of the invention encodes an antibiotic resistance gene and the expression of the reporter gene is detected by cultivating the cells in a medium containing the corresponding antibiotic.

Typically, the activity of the reporter gene product can be determined by spectrophotometric, colorimetric, fluorimetric, or luminometric methods.

According to a particular embodiment, at least one of the one or more reporter genes comprises a coding sequence of *HIS3,* which encodes a factor essential in the synthesis of Histidine.

In particular embodiments, engineered cells of the invention are further characterized in that they have been modified either genetically or chemically to facilitate the uptake of agents, compounds or chemical signals.

The engineered cell of the invention are engineered yeasts. More particularly, the engineered yeast cell is of the order of the Saccharomycetales, preferably *Saccharomyces cerevisiae.* More particularly, the invention relates to strains of yeast which comprise one or more reporter gene(s) under transcriptional control of a promoter of a heat shock protein and one or more gene(s) encoding one or more protein(s) prone to misfolding and are characterized by spontaneous misfolding of one or more proteins prone to protein misfolding as a result of a spontaneous mutation, or are characterized by inducible misfolding.

The invention includes also the progeny and all subsequent generations of the engineered cells into which the DNA sequence(s) were introduced.

In a particular aspect of the invention, engineered cells according to particular embodiments described above are used to determine the effect on protein misfolding of a protein or peptide encoded by a cDNA. In this aspect the engineered cell of the invention is further transformed with the relevant cDNA. More particularly, a culture of the engineered cells of the invention is provided comprising one or more reporter genes and one or more genes encoding a protein prone to protein misfolding which further comprises an expression vector comprising a cDNA.

A further aspect of the present invention provides methods for generating the engineered cells of the present invention, which methods comprise, transforming a cell with at least one gene encoding a reporter gene responsive to protein misfolding. The methods optionally further comprise transforming the cell with at least one gene encoding a protein prone to protein misfolding.

In a particular embodiment, methods are provided for producing an engineered yeast cell of particular use in monitoring protein misfolding, which method comprises the steps of providing an engineered cell of a yeast strain with one or more reporter gene(s) under transcriptional control of a promoter of a heat shock protein and one or more genes encoding one or more proteins prone to misfolding; cultivating the yeast strain so obtained under conditions which allow spontaneous mutations and selection of a yeast cell wherein the one or more reporter gene(s) are expressed at higher levels, compared to the strain prior to the cultivation. In a particular embodiment, the one or more reporter gene(s) are expressed as function of the presence of the misfolding-prone protein.

According to a further particular embodiment, methods for producing an engineered yeast cell of the invention comprise the steps of: transforming cells of a yeast strain with one or more reporter gene(s) under transcriptional control of a promoter of a heat shock protein; and transforming the cells of the yeast strain so obtained with at least one gene encoding one or more proteins prone to misfolding. Alternatively, the one or more genes encoding one or more proteins prone to misfolding are introduced into the yeast strain prior to the one or more reporter genes. In yet a further embodiment, both the one or more reporter genes and the one or more genes encoding one or more proteins prone to misfolding are introduced into the yeast strain simultaneously.

A further aspect of the invention provides for the use of engineered cells described above for monitoring protein misfolding. Additionally or alternatively the cells according to particular embodiments of the invention are used as a model for (a) disease(s) associated with aberrant protein folding. Typical diseases envisaged in the context of the present invention, are neurodegenerative diseases such as but not limited to Alzheimer's, Parkinson's or Huntington's disease.

Yet a further aspect of the invention provides methods for screening compounds for their ability to directly or indirectly affect protein misfolding. The methods can be provided as an assay, automated assay or high throughput screening assay. Screening methods provided in the invention comprise the steps of providing engineered yeast cells according to the present invention described above, the cells being characterized by either spontaneous or inducible misfolding of the one or more protein(s) prone to misfolding; contacting the cells with the one or more compounds by adding the one or more compounds to the cells or their medium; and detecting the change in activity of the reporter gene product in the cells to determine the effect of the one or more compounds on the occurrence of protein misfolding in the engineered cell.

The nature of the detection step of screening methods according to particular embodiments of the present invention is determined by the nature of the reporter gene. Proliferation of the cells can be quantified, for instance by measuring optical density of the cell culture. Alternatively, the reporter gene can be a gene that encodes a signal which can be detected by an optical method.

Specific embodiments of methods provided herein further comprise the steps of modifying the engineered cells described herein either genetically or chemically to facilitate the uptake of agents, compounds or chemical signals.

Specific embodiments of screening methods of the present invention encompass the use of engineered cells of the invention in the screening of cDNA's e.g. as part of a DNA library, to identify whether the cDNAs encode proteins or peptides that directly or indirectly affect protein misfolding. Methods according to this embodiment comprise the steps of providing engineered cells according to particular embodiments of the invention as described above, the cells being characterized by either spontaneous or inducible misfolding of the one or more protein(s) prone to misfolding; transforming a culture of the engineered cells with an expression cDNA library; and detecting the activity of the reporter gene product in each of the transformed cells to determine the occurrence of protein misfolding in the transformed cell.

In particular embodiments screening methods provided in the present invention, encompasses cultivating an engineered yeast cell comprising one or more reporter gene(s) under transcriptional control of a promoter of a heat shock protein and one or more gene(s) encoding one or more protein(s) prone to misfolding, and selecting a cell wherein said one or more reporter gene(s) are expressed at higher levels compared to the original strain before the selection.

According to specific embodiments of methods of the invention, this step is done by providing an engineered yeast cell comprising one or more reporter gene(s) under transcriptional control of a promoter of a heat shock protein and one or more gene(s) encoding one or more protein(s) prone to misfolding, and contacting the yeast cell with external factors to induce misfolding of the protein prone to misfolding.

Most particularly, at least one of said protein(s) prone to misfolding is α-synuclein and the external factor is paraquat.

According to particular embodiments screening methods of the present invention further comprise, prior to detecting the activity of the reporter gene product, the culturing of the engineered cells in an appropriate medium to allow the detection of the activity of the reporter gene product.

According to further particular embodiments, screening methods of the invention further comprise the step of comparing the detection of expression of the reporter gene upon adding the compound or upon expression of the relevant cDNA, with an appropriate positive and/or negative control, so as to determine the effect of the compound or the expression of the cDNA on protein misfolding in the cell.

The easiness of the methods provided herein to detect misfolding strongly facilitates their applicability in high-throughput screens (e.g. of chemical libraries or cDNA libraries). An important advantage of methods presented herein is that it is not necessary for the biological processes underlying abnormal protein folding to be fully elucidated, as compounds interfering with each step of the pathological process leading to protein misfolding and/or aggregation can be identified. Indeed, present methods allow identifying therapeutic compounds or agents which may be regulators of protein folding and/or inhibitors of protein aggregation and/or preventors and/or inhibitors of fibril formation, or possibly even those having an entirely different and possibly unknown mechanism of action.

### BRIEF DESCRIPTION OF THE FIGURES

Figure 1 - Schematic representation of the molecular mechanisms underlying the detection of disease-related protein misfolding according to an embodiment of the present invention. Protein misfolding and/or aggregation give rise to several pathological protein conformations (A). This leads to activation of specific transcription factors (such as Hsf1), which in turn initiate transcription from target genes under control of specific promoters to which these transcription factors bind (B). By placing a reporter gene under control of such promoter, the formation of non-native protein conformations in the cell results in specific increased expression of the reporter gene product, which can be detected.

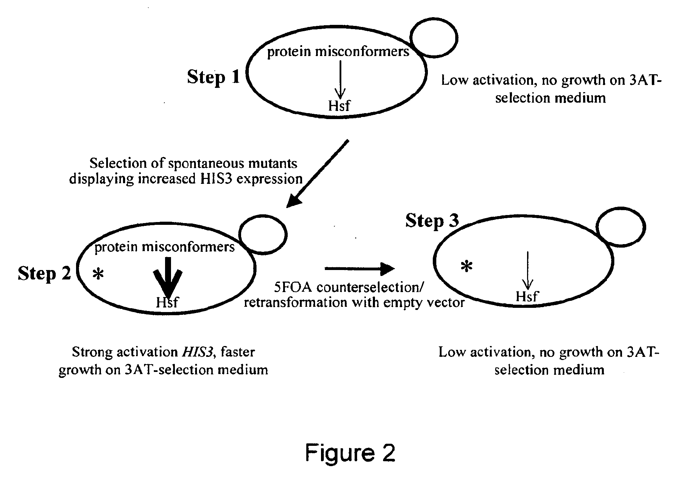

Figure 2 - Schematic representation of the selection procedure to isolate cell lines in which a protein prone to protein misfolding activates Hsf1-driven *pSSA3-HIS3* reporter gene expression according to an embodiment of the invention.

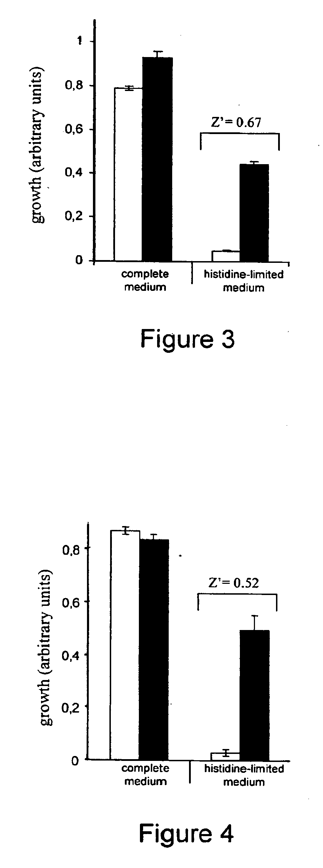

Figure 3 - Comparison of the growth of a yeast strain (H9) comprising a *HIS3* reporter gene (*pSSA3-HIS3*) transformed with an empty vector (open bars) or with a construct encoding human tau (solid bars) on standard complete medium and histidine-limited medium, according to an embodiment of the invention. Expression of the gene encoding human tau activates a Hsf1-regulated promoter and expression of the *HIS3* gene, as demonstrated by increased growth on histidine-limited medium. No significant difference in growth was observed on complete medium.
Figure 4 - Comparison of the growth of a yeast strain (B11) comprising a *HIS3* reporter gene (*pSSA3-HIS3*) transformed with an empty vector (open bars) or with a construct encoding human α-synuclein (solid bars) on standard complete medium and histidine-limited medium, according to an embodiment of the invention. Expression of the gene encoding human α-synuclein activates a Hsf1-regulated promoter and consequently expression of the *HIS3* gene, as demonstrated by increased growth on histidine-limited medium. No significant difference in growth was observed on complete medium.

### DETAILED DESCRIPTION OF THE INVENTION

### Definitions

The term "native protein" as used herein, refers to the protein or peptide as it occurs in the living cell or organism in natural, non-disease conditions. This terms is intended to distinguish the protein as it is normally present in the organism, from modified proteins, such as mutants, modifications of side groups or substituents and modifications of secondary structure or tertiary structure, which can lead to protein misfolding. Accordingly, the native conformation of the native protein refers to the three-dimensional structure of the protein as present in its natural (non-diseased) cellular environment, and in which it is biologically active.

The term "protein misfolding" as used herein refers to the process whereby a protein, peptide or a fragment or mutant thereof adopts (a) alternative, possibly stable conformation(s) different from the native conformation of the native protein or peptide whereby this alternative conformation remains present in the cells at a level which is higher than background levels. Thus, a cell characterized by protein misfolding is understood to refer to a cell in which abnormal misfolding of one or more proteins occurs independent of the occasional misfolding associated with the normal folding process, which naturally occurs in cells, for which correction mechanisms are in place and which usually remains undetected. Protein misfolding can occur by aberrant folding of an unfolded polypeptide chain or by unfolding, partial unfolding or denaturation of a natively folded protein.

Protein misfolding as used herein can be either spontaneous or inducible. The term "spontaneous", when used to refer to protein misfolding, is intended to refer to protein misfolding occurring within the cell without the requirement of external factors, i.e. as a result of one or more aberrant internal mechanisms within the cell. The term "inducible", when used herein to refer to protein misfolding, is intended to refer to protein misfolding occurring within a cell as a result of external factors capable of inducing misfolding. In the context of the present invention, a cell characterized by inducible protein misfolding implies that misfolding of essentially only the one or more proteins prone to misfolding can be induced by contacting the cell with an external factor (and thus does not refer to the feature of general protein misfolding which can be induced in most cells).

As used herein, "protein aggregation" refers to the accumulation, oligomerization, fibrillization or aggregation of two or more, hetero- or homomeric proteins or peptides, or fragments or mutants thereof. "Detrimental protein aggregation" is protein aggregation which compromises the functioning and/or viability of the cell or organism in which it occurs.

A "protein prone to misfolding" as used herein refers to a protein, peptide or a fragment which has at least one conformation, different from the native conformation of the native protein or peptide in a living cell, which can lead to protein aggregation either because the aberrantly folded protein has a stable or de facto (e.g. kinetically) stabilized conformation, or because the misfolding of the protein occurs at higher levels than background levels, resulting in higher levels of misfolded protein in the cell. The proteins prone to misfolding in the present invention are the amyloidogenic proteins mentioned below and serpins, poly(A) binding protein 2, low-density lipoprotein receptor, apolipoprotein B100, cochlin, RET, myelin protein 22/0, short-chain acyl CoA dehydrogenase, cystic fibrosis transmembrane conductance regulator, HERG, phenylalanine hydroxylase, fibrillin, procollagen, collagen, haemoglobin, ATPase ATP7B and β-hexosaminidase tyrosinase.

An "amyloidogenic protein" as used herein refers to a subtype of proteins prone to protein misfolding, which are capable of forming amyloid or amyloid-like fibrils, aggregates or deposits, and refers to a protein selected from the group consisting of amyloid precursor protein, tau, α-synuclein, huntingtin, ADan peptide, ABri peptide, amyloid β, prion protein, ataxins, superoxide dismutase, cystatin C, atrophin 1, androgen receptor, TATA box-binding protein, transthyretin, serum amyloid A, Ig light chains, β2-microglobulin, apolipoprotein A-1, gelsolin, pro- islet amyloid polypeptide, procalcitonin, lysozyme, insulin, fibrinogen α-chain, atrial natriuretic factor, surfactant protein C, rhodopsin, γ-crystallins, lactoferrin, βig-h3, kerato-epithelin, corneodesmosin, p53, lactadherin and prolactin.

The terms "promoter responsive to protein misfolding" as used herein refers to a promoter of a heat shock protein which is directly or indirectly activated to drive expression of the coding sequence operably linked thereto, by the occurrence of misfolded proteins in the cellular environment. Typically, the signal which activates the promoter is not the misfolded protein *per se* but a factor which is modulated (typically activated) by misfolded proteins, which upon activation, binds to the promoter and activates transcription.

The term "transgene" as used herein refers to any DNA sequence comprising a coding sequence and a promoter, which has been introduced into a cell, and is part of the genome of the resulting organism (i.e. either stably integrated or as a stable extrachromosomal element). A transgene may be a foreign DNA sequence, i.e. a DNA sequence encoding a protein not endogenously present in that cell, or a DNA sequence that is endogenous to the cell but is not normally present in that location in the genome, or is not normally under control of the same regulatory sequences. The transgene may have been introduced directly into the cell or may have been introduced into an earlier generation of the cell.

Included within this definition is a transgene created by providing an RNA sequence which is reverse transcribed into DNA and then incorporated into the genome and (constitutively expressed) of antisense.

As used herein, the term "minigene" refers to a heterologous gene construct wherein one or more nonessential segments of a gene are deleted with respect to the naturally occurring gene.

The term "engineered cell" is used herein to refer to a cell, having a transgene present as an extrachromosomal element or stably integrated into its germ line DNA (i.e. in the genomic DNA).

The term "compound" is used herein to refer to a molecule of any type including a chemical compound, a peptide or nucleotide (e.g. antisense) sequence, a mixture of chemical compounds, a biological macromolecule, or an extract of biological material such as bacteria, plants, fungi, or animal (particularly mammalian) cells or tissues.

It is to be understood that, in the context of the invention, when a singular noun is used, the plural is also incorporated, and that 'a' or 'an' may mean more than one. Similarly, when plural is used, the singular is also encompassed, unless explicitly stated otherwise.

It is contemplated that compositions and steps discussed in the context of one embodiment or aspect of the invention may be employed with respect to other embodiments or aspects discussed herein.

The present invention is based on the observation that the use of a cellular system comprising a reporter gene responsive to protein misfolding in the cells allows the rapid and sensitive detection of the effect of compounds on any aspect of protein misfolding. Based thereon, the invention provides tools and methods for the identification of compounds affecting protein misfolding.

In a first aspect of the invention, engineered yeast cells are provided which are responsive to protein misfolding and resulting aggregation. More particularly, the invention provides yeast cells comprising one or more reporter genes under transcriptional control of a promoter of a heat shock protein. According to the present invention, the engineered yeast cell lines further comprise one or more genes encoding a protein prone to protein misfolding. As a result of misfolding and eventually aggregation of the protein prone to protein misfolding, the reporter gene is activated resulting in a signal that can be detected directly or indirectly.

The cells envisaged in the context of the present invention are yeast cells.Further disclosed herein are cells in the form of a cell line that is suitable for continuous culture, either in suspension, semi-suspension, monolayer, and/or as colonies on solid or semi-solid medium. For example, such cells are from an immortalized cell line. For example, the cell line originates from mammals. cell lines in which hsf1 is naturally expressed are preferred. Other examples of cells are neural cells, such as human neural cells. Further disclosed are eukaryotic cell lines which are particularly amenable to mutations, such as the mouse fibroblast cell line NIH3T3 or DNA-repair deficient cell lines.

The cells of the present invention are engineered yeast cells, as this organism combines ease of manipulation with the possibility of cost-effective screening. A cell culture obtained after cultivation under appropriate conditions and selection for a certain phenotype will also be referred to herein as a "strain".

A particularly suitable yeast strain for use in context of the invention is *Saccharomyces cerevisiae,* but the use of any yeast strain is envisaged. Some other, non-limiting examples of yeast cell strains that are envisaged include *Schizosaccharomyces pombe, Saccharomyces kluyveri, Saccharomyces uvae, Saccharomyces uvarum, Kluyveromyces lactis, Hansenula polymorpha, Pichia pastoris, Pichia kluyveri, Pichia methanolica, Yarrowia lipolytica, Candida sp., Candida utilis, Candida cacaoi, Geotrichum sp.* and *Geotrichum fermentans.*

The present invention provides for cellular systems in which the misfolding of one or more proteins within the cell is made detectable using one or more reporter genes.

According to the present invention, the one or more reporter genes present in the engineered cells of the present invention are under transcriptional control of a promoter of a heat shock protein. Typically the reporter gene responsive to protein misfolding is a transgene, which has been stably introduced into the genome of a cell.

In response to heat shock or protein misfolding, a transcription factor called 'heat shock factor' (HSF) binds to the heat shock elements (HSEs) present in the promoter region of heat shock genes. These HSEs are found as three repeats of a 5-nucleotide {nGAAn} module, arranged in alternating orientation and present upstream of all heat shock genes. Thus, placing a coding sequence under control of a promoter comprising a HSE, will result in expression induced by heat-shock or protein misfolding. The activation of HSF is independent of the nature of the protein being misfolded, so that HSF-driven promoters can be used to detect the misfolding of any protein in the context of the present invention.

Different genes have been described in the art to be induced upon protein misfolding and/or aggregation. Particularly, in yeast, it has been shown that expression of over 200 genes is specifically induced by protein misfolding, most of which are also induced upon temperature upshift (Trotter et al., J Biol Chem. 2002 277(47): 44817-25, see Table S1 in the supplemental material). Most particularly, suitable promoters include those known or suspected to be to be part of the HSE regulon in yeast, e.g. *HSP12, HSP104, HSP42, HSP78, HSP30, HSP82, HSP26, SSA3, SSA4* and *SSE2.* Homologous HSF-reactive genes have been described in a number of organisms, including *Drosophila* and humans, and appear to be highly conserved. Three different HSFs have been identified in humans, Hsf1, Hsf2 and Hsf4.

It has been demonstrated that expression of a small subset of genes in yeast is strongly induced by protein misfolding, but not by temperature upshift. The mechanism does not involve activation of heat shock factor. These genes include *CUP1-1, CUP1-2, MAG1, TRX2, MSS2* and *UBC6* (Trotter et al., J Biol Chem. 2002 277(47): 44817-25, particularly Table S1 in the supplemental material). It is envisaged that the promoters of these genes are also suitable for use in the context of the present invention.

According to particular embodiments, the promoter used to drive expression of at least one of the one or more reporter genes in the engineered cell of the invention is a Hsf1-reactive promoter, such as the yeast *SSA3* promoter, the human *hsp70* promoter or an E. coli σ³² controlled promoter. Alternatively, a Hsf2 or Hsf4 reactive promoter can be used.

The nature of the reporter genes used in the context of the present invention is not critical. In principle, any DNA sequence encoding a protein the expression of which can be specifically determined is suitable. The detection of the expression of the reporter gene can for instance be by an optical method, such as spectrophotometric, colorimetric, fluorimetric, or luminometric methods. This detection can be a direct detection of the gene product or can be the result of a reaction of the gene product with one or more compounds present in the cells or added to the medium of the cells. According to one embodiment of the invention, the reporter gene encodes an enzyme, which acts on a substrate added to the medium of the cells, and the reaction product can be optically detected. Typical examples of reporter genes the expression of which can be directly or indirectly detected by optical methods include, but are not limited to, luciferase, β-galactosidase, β-glucuronidase (gus), alkaline phosphatase, lacZ, chloramphenicol acetyltransferase, green fluorescent protein, cyan fluorescent protein, yellow fluorescent protein, dihydrofolate reductase (DHFR) and horseradish peroxidase.

According to a particular embodiment, the engineered cells of the present invention comprise at least one reporter gene encoding a protein that positively or negatively affects growth and/or proliferation of the cells. The effect of the reporter gene product on growth and/or proliferation of the cells in which it is expressed can be achieved directly by the activity of the reporter gene product. Alternatively, the effect of the reporter gene product on the growth of the cell is obtained when subjecting the cells to specific conditions, such as cultivating the cells in specific media or addition to or removal from the medium of specific factors or agents. In a specific embodiment, the cells of the invention comprise a reporter gene encoding a protein that allows synthesis of an essential amino acid. More specifically, the present invention provides cells comprising a reporter gene comprising the coding sequence of *HIS3,* the gene product of which is required for the synthesis of histidine. Expression of this reporter gene by the cells can be determined by placing the cells on histidine-limited medium. Activation of the reporter gene allows the cells to grow faster on histidine-limited medium relative to cells in which the reporter gene is not activated. On standard medium comprising histidine, reporter gene expression does not significantly affect viability or growth of the cells.

According to a specific embodiment, the effect of the reporter gene product on growth and/or proliferation of the engineered cells of the present invention is further enhanced by supplementing the growth medium with one or more substances that enhance the effect of the reporter gene product on growth and/or proliferation of the cell. Such substances are referred to as "modulators of reporter gene activity". A typical example of a modulator of reporter gene activity is 3-amino-1,2,4-triazole (3AT), which modulates *HIS3* reporter gene activity by inhibiting the gene product imidazoleglycerolphosphate dehydratase. The effects on growth and/or proliferation by differential *HIS3* reporter gene expression is enhanced by addition of 3AT to SC-HIS medium.

According to yet another embodiment, the reporter gene product confers a resistance to the cell to an agent or environmental factor, which can be detected upon contacting the cell with the relevant agent or environmental factor. More particularly, in one embodiment the reporter gene confers e.g. antibiotic resistance, and expression of the reporter gene can be detected upon supplementing the medium with an appropriate antibiotic. Suitable antibiotic resistance genes and corresponding antibiotics have been described and include e.g. *bla* (ampicillin); genes encoding aminoglycoside modifying enzymes e.g. *kan, nptII* or *nptIII* (kanamycin, neomycin, gentamycin B, geneticin, G418); *aadA* (streptinomycin), *tetA* (tetracyclin), *cyh2* (cycloheximide), *nat* (nourseothricin), *hph* (hygromycin B), *aur1-c* (aureobasidin A), *ble* (phleomycin), *bar* (bialaphos), *cat* (chloramphenicol), *pac* and *pmh* (both suited for selection with puromycin).

According to yet another embodiment, the reporter gene encodes a metabolite, and its expression is detected by adding the corresponding antimetabolite to the cell. An antimetabolite interferes with normal biochemical reactions of the cell, due to its structure which is similar to that of a natural metabolite. When placed in a medium comprising antimetabolite, expression of a reporter gene encoding the corresponding metabolite will help the cell survive. Non-limiting examples of antimetabolites include canavanine, selenomethionine, norleucine, ethionine, 2,5-dihydrophenylalanine, m-fluorophenylalanine, o-fluorophenylalanine, p-fluorophenylalanine, azetidine-2-carboxylate and methotrexate.

Alternatively, the reporter gene encodes a mutated enzyme which confers resistance to an antimetabolite, because, contrary to the native enzyme, the mutant enzyme does not catalyze reactions with the antimetabolite. For instance, a mutation in the S-adenosylmethionine synthetase encoding gene (e.g. *S. cerevisiae SAM1* or *SAM2)* confers resistance to ethionine.

According to yet another embodiment, the reporter gene product confers a sensitivity to the cell to an agent or environmental factor, which can be detected upon contacting the cell with the relevant agent or environmental factor. More particularly, in one embodiment the reporter gene confers sensitivity by metabolizing an appropriate substrate to a cytotoxic product (counterselection), and expression of the reporter gene can be detected upon supplementing the medium with said substrate. Suitable counterselection genes and corresponding substrates have been described and include: the gene encoding orotidine-5'-phosphate decarboxylase (e.g. *S. cerevisiae URA3)* with 5-fluorootic acid (5FOA); the gene encoding L-aminoadipate-semialdehyde dehydrogenase (e.g. *S. cerevisiae LYS2)* with α-amino adipate; the gene encoding phosphoribosylanthranilate isomerase (e.g. *S. cerevisiae TRP1)* with 5-fluoroanthranilic acid (5FAA); and the gene encoding O-acetylhomoserine aminocarboxypropyltransferase (e.g. *S. cerevisiae MET17)* with methylmercury.

According to the present invention, cells are provided comprising a reporter construct susceptible to protein misfolding and resulting aggregation and further comprising one or more genes encoding proteins prone to protein misfolding.

The nature of the protein prone to protein misfolding present in the cells is determined by the application of the cells but is not critical to the present invention. Moreover, in view of the fact that the reactivity of most of the promoters reactive to protein misfolding is not protein-specific, the reporter genes described herein can be used to detect the misfolding of any protein of interest.

In general, proteins known to be prone to protein misfolding do not share a high structural or sequence homology. However, despite the significant differences between the proteins in their normally folded and non-aggregated state, once misfolded, the proteins share common features, and the mechanism by which they are involved in the pathology of conformational diseases appears to be the same.

Upon aberrant folding of the protein, the proteins will show a propensity to self-associate or aggregate with each other, which in many cases is detrimental to the cell or organism wherein they occur. These detrimental protein aggregates almost always contain very typical structural features: β-sheets and/or fibril-like structures and/or highly hydrophobic domains. A detrimental protein aggregate may be deposited in bodies, inclusions or plaques, the presence of which *in vivo* is often indicative of disease.

According to the present invention, cultivation of a cell comprising one or more genes encoding one or more protein(s) prone to protein misfolding under conditions which either induce misfolding or allow spontaneous misfolding to occur, results in misfolding and optionally aggregation, which can be detected rapidly and sensitively based on the activation of reporter gene expression.

According to the present invention, engineered cells are provided which are characterized by either spontaneous or inducible misfolding of one or more proteins prone to misfolding.

Spontaneous misfolding occurring in the engineered cells of the invention can be the result of one or more modifications in the control and regulation mechanism of protein folding in the cell. Indeed, while limited protein misfolding naturally occurs in cells as a side-effect of the natural folding mechanisms, the resulting limited amount of misfolded protein is removed from the cell by internal quality control mechanisms within the cell, which minimize the effects of low level aberrant protein misfolding. However, factors impairing the normal activity of these repair mechanisms (e.g. one or more mutations in one or more gene encoding proteins involved in the folding quality control mechanism) will result in detectable misfolding within the cell. This is observed as a natural process, e.g. in ageing cells. Additionally or alternatively, spontaneous misfolding can be the result of increased rate of misfolding or an increased stability of the misfolded protein (which in a way can also be considered as a factor "impairing the normal activity of repair mechanism"), as a result, e.g. of mutations in the protein or in the factors involved in the natural protein folding mechanisms.

According to one embodiment of the invention, the engineered cells of the invention characterized by spontaneous misfolding of the one or more proteins prone to misfolding are obtained by cultivating the cells comprising the one or more reporter genes and the one or more genes encoding one or more proteins prone to misfolding under conditions which allow or induce the misfolding of the protein prone to protein misfolding. More particularly, upon cultivation of a yeast strain expressing the protein prone to protein misfolding, genomic mutations will spontaneously occur, a number of which will result in the misfolding of the one or more proteins prone to misfolding. Such mutations occur in yeast when grown under standard cultivation conditions (such as those described by Rose et al. Methods in yeast genetics, a laboratory course manual., Cold Spring Harbor Laboratory Press, Cold Spring harbor, N. Y. (1990)). The standard media used for the cultivation of yeast in the context of the present invention can be further supplemented with or depleted of one or more specific compounds, as required for the detection of the reporter gene activity. Indeed the present invention, a mutation in a strain resulting in the misfolding of the protein prone to misfolding can be identified based on the expression of the reporter gene, whereby cultivation and detection is optimally performed in the same medium. Mutagenesis can also be induced by subjecting the cells to mutation inducing factors such as, but not limited to, radiation, chemicals (Ethylmethane Sulphonate-EMS), transposon tagging etc..

Additionally or alternatively, according to particular embodiments of the present invention, the engineered cells are characterized by inducible misfolding of the one or more proteins prone to misfolding. This can be achieved by the presence in the cell of a protein prone to misfolding for which misfolding can be specifically induced by subjecting the cells to particular agents or conditions. These agents or conditions may boost expression of the gene of interest. A well-known example of such an external agent is paraquat, which causes upregulation and aggregation of α-synuclein (Manning-Bog et al., J Biol Chem. 2002; 277(3): 1641-4). Rotenone or MPTP can be used in a similar way as paraquat. Additional or alternative toxicity inducing agents can be envisaged such as, but not limited to a carbon source, nitrogen source, salt, metal, azauracil, aurintrincarboxylic bleomycin, brefeldin A, camptothecin, chlorambucil, ethidium bromide, formamide, GuHCI, hydroxyurea, menadione, vanadate. In some embodiments, the carbon source is arabinose, ethanol, or glycerol, while in other embodiments, a nitrogen source is urea. In further embodiments, the toxicity inducing agent is a salt or metal, such as CaCl₂, CoCl₂, CsCl, or iron, magnesium, RbCl, or SrCl₂.

An additional or alternative mechanism for inducing misfolding of a protein prone to protein misfolding is by contacting the cell with already aggregated species. It has been demonstrated that the presence of abnormally folded protein can cause misfolding of the native protein.

The present invention envisages embodiments wherein one or more types of misfolding are present, i.e. the misfolding can be either spontaneous or induced by one or more factors or can be both spontaneous and in addition induced by an external factor.

While the nature of the protein prone to misfolding is not critical for the methods and assays of the present invention, specific embodiments of the invention are characterized in that at least one of the encoded proteins prone to protein misfolding is an amyloidogenic protein or mutant thereof. The amyloidogenic proteins normally occur as soluble monomers, but upon misfolding, start aggregating in soluble oligomers. Further aggregation leads to formation of insoluble aggregates (e.g. amyloid or amyloid-like fibrils, eventually resulting in amyloid or amyloid-like deposits). The amyloid or amyloid-like fibrils formed as a result of detrimental protein aggregation possess several characteristic features, including: they display a wound and predominantly unbranched morphology, their core comprises a cross-β structure wherein the strands of the β-sheets run perpendicular to the main fibril axis (which can be assessed by e.g. spectroscopy or X-ray diffraction), they have distinctive dye-binding properties (e.g. exhibiting so-called apple-green or red-green birefringence under polarized light after staining with Congo red; fluorescent staining with thioflavin S or thioflavin T) and are relatively resistant to proteolysis. One or more of these features may be used to assess whether a fibril is amyloid-like. The protein misfolding of amyloidogenic proteins ultimately results in the formation of amyloid-like deposits, which may contain one or more than one amyloidogenic protein (e.g. amyloid β and tau in Alzheimer's disease).

In the context of the present invention, amyloidogenic proteins are amyloid precursor protein, tau, α-synuclein, huntingtin, ADan peptide, ABri peptide, amyloid β, prion protein, ataxins, superoxide dismutase, cystatin C, atrophin 1, androgen receptor, TATA box-binding protein, transthyretin, serum amyloid A, Ig light chains, β2-microglobulin, apolipoprotein A-1, gelsolin, pro-islet amyloid polypeptide, procalcitonin, lysozyme, insulin, fibrinogen α-chain, atrial natriuretic factor, surfactant protein C, rhodopsin, γ-crystallins, lactoferrin, βig-h3, kerato-epithelin, corneodesmosin, p53, lactadherin and prolactin. A specific embodiment of the present invention relates to engineered cells comprising a mutant form of tau or α-synuclein, which upon expression in the engineered cells, results in protein misfolding.

Although amyloidogenic proteins constitute a particular class of proteins, other proteins prone to protein misfolding, which are not identified as amyloidogenic, or of which the classification is unsure, are serpins (including α1-antitrypsin, neuroserpin, antithrombin, α1-antichymotrypsin, complement 1 inhibitor), poly(A) binding protein 2, low-density lipoprotein receptor, apolipoprotein B100, cochlin, RET, myelin protein 22/0, short-chain acyl CoA dehydrogenase, cystic fibrosis transmembrane conductance regulator, HERG (also known as KCNH2), phenylalanine hydroxylase, fibrillin, procollagen, collagen, haemoglobin, ATPase ATP7B, β-hexosaminidase and tyrosinase.

According to one embodiment, at least one of the one or more genes encoding the protein prone to protein misfolding is an endogenous gene, under control of its natural promoter. According to an alternative embodiment, however, at least one of the one or more genes encoding the protein prone to protein misfolding is a transgene. According to a particular embodiment, one or more of the transgenes encoding a protein prone to protein misfolding present in the cells of the present invention comprises an endogenous sequence encoding a protein prone to protein misfolding, under the control of a promoter which is transgenic, i.e. a promoter which does not naturally control transcription of that sequence in the cell. This can be an endogenous promoter to the cell of the invention, or can be a foreign promoter.

According to further embodiments, one or more of the transgenes encoding a protein prone to protein misfolding present in the cells of the present invention comprises a transgenic coding sequence encoding a protein prone to protein misfolding, under the control of an endogenous, non-transgenic promoter.

According to a particular embodiment however, both the promoter and the DNA sequence encoding the protein prone to misfolding are transgenic, i.e. do not naturally occur in the genome of the cell in that location. The transgenic promoter and coding sequence can both be either endogenous or foreign to the cell. Most particularly, the transgenic promoter is endogenous to the cell, while the coding sequence is foreign to the cell. For instance in yeast, a yeast promoter is used to drive the expression of a mammalian protein, more particularly an amyloidogenic protein. Alternatively, both the promoter and the coding sequence of the protein prone to misfolding are foreign to the cell. Typically, in the context of screening of therapeutic compounds, a protein of human origin will be used, to mimic the misfolding occurring in the disease condition. According to one embodiment, the transgenic yeast cells comprise at least one DNA sequence encoding a protein prone to protein misfolding from human origin. More particularly, the DNA sequence encoding a protein prone to detrimental protein aggregation is a DNA sequence encoding a human amyloidogenic protein, such as human tau (Genbank gi:82534350, gi:82533198, gi:82533197 and gi:82534391) or human α-synuclein (Genbank gi:6806896 and gi:680697). According to yet a further particular embodiment of the invention, the transgenic cells of the invention comprise a transgene encoding a protein prone to detrimental protein aggregation, which transgene comprises a human gene encoding an amyloidogenic protein, such as the human tau gene (Entrez GeneID: 4137) or the human α-synuclein gene (Entrez GeneID: 6622).

Different promoters are considered suitable for driving the expression of the one or more genes encoding a protein prone to protein misfolding in the context of the present invention, as long as expression in the cells of the invention is ensured. For the transcriptional control of genes encoding proteins prone to protein misfolding, both constitutive and inducible promoters are envisaged in the context of the present invention. According to a particular embodiment, the promoter is a promoter which is endogenous to the cell.

Typical examples include, but are not limited to inducible promoters (e.g. *IPTG* promoter, *Tet* promoter, *GAL1* promoter), and synthetic promoters (typically containing specific responsive elements).

According to a particular embodiment of the invention, at least one of the genes encoding a protein prone to protein misfolding is a minigene. A minigene is a heterologous gene construct wherein one or more nonessential segments of a gene are deleted with respect to the naturally occurring gene. Typically, deleted segments are intronic sequences of at least about 100 basepairs to several kilobases, and may span up to several tens of kilobases or more.

Typically, in minigenes, intronic sequences that do not encompass essential regulatory elements are deleted. Frequently, if convenient restriction sites border a nonessential intronic sequence of a cloned gene sequence, a deletion of the intronic sequence may be produced by: (1) digesting the cloned DNA with the appropriate restriction enzymes, (2) separating the restriction fragments (e.g., by electrophoresis), (3) isolating the restriction fragments encompassing the essential exons and regulatory elements, and (4) ligating the isolated restriction fragments to form a minigene wherein the exons are in the same linear order as is present in the germline copy of the naturally-occurring gene.

Alternate methods for producing a minigene will be apparent to those of skill in the art (e.g., ligation of partial genomic clones, which encompass essential exons but which lack portions of intronic sequence). Most typically, the gene segments comprising a minigene will be arranged in the same linear order as is present in the germline gene, however, this will not always be the case. Some desired regulatory elements (e.g., enhancers, silencers) may be relatively position-insensitive, so that the regulatory element will function correctly even if positioned differently in a minigene than in the corresponding germline gene. For example, an enhancer may be located at a different distance from a promoter, in a different orientation, and/or in a different linear order. For example, an enhancer that is located 3' to a promoter in germline configuration might be located 5' to the promoter in a minigene. Similarly, some genes may have exons, which are alternatively spliced, at the RNA level, and thus a minigene may have fewer exons and/or exons in a different linear order than the corresponding germline gene and still encode a functional gene product.

According to one embodiment, the engineered cells of the present invention comprise one gene encoding a protein prone to misfolding and eventual aggregation. Typically, the protein selected is a protein known to be involved in detrimental protein aggregation and/or misfolding in the disease of interest. Alternatively, however, the cells comprise more than one gene encoding a protein prone to protein misfolding and/or misfolding. The more than one gene can both comprise the same coding sequence or can comprise different coding sequences.

The cells of the present invention are of use in the identification of compounds and conditions capable of affecting protein misfolding. In this context it can be of interest to ensure that the effect observed on the cells of the invention is specifically associated with the one or more genes prone to protein misfolding. To this effect, the invention further provides engineered cells comprising in addition to the one or more reporter genes and one or more transgenes encoding one or more proteins prone to misfolding, a marker for negative selection (counter selection) as part of the one or more transgenes, which provides a control to determine that the observed effect is directly linked to the expression of the protein prone to misfolding, e.g. a control on whether or not the observed effect is associated with the expression of one or more of the transgenes encoding one or more proteins prone to misfolding. Typical examples of counter-selectable genes which can be used as negative selection markers and their corresponding substrates include, but are not limited to:
- a gene encoding orotidine-5'-phosphate decarboxylase (e.g. S. cerevisiae URA3) with 5-fluorootic acid;
- a gene encoding L-aminoadipate-semialdehyde dehydrogenase (e.g. S. cerevisiae LYS2) with α-amino adipate;
- a gene encoding phosphoribosylanthranilate isomerase (e.g. S. cerevisiae TRP1) with 5-fluoroanthranilic acid; and
- a gene encoding homoserine transacetylase (e.g. S. cerevisiae MET2), O-acetylhomoserine aminocarboxypropyltransferase (e.g. S. cerevisiae MET17) or O-acetylhomoserine sulfhydrylase (e.g. S. cerevisiae MET25) with methylmercury.

As indicated above, particular embodiments of the invention provides cells wherein, the protein prone to misfolding is an endogenous protein. In methods making use of these cells, counterselection can be done by knockout of the gene encoding said protein, or by inhibiting expression of its gene product, e.g. by RNAi. Thus, the invention further provides for engineered yeast cells comprising in addition to the one or more reporter genes and an endogenous gene encoding a protein prone to misfolding, a gene which inhibits expression of the endogenous gene encoding the protein prone to misfolding.

According to a particular embodiment, the engineered cells used in the invention are further modified, either genetically or chemically, to facilitate uptake of agents, compounds or chemical signals. This is of interest for use of the cells in the context of compound screening, to ensure uptake of the compounds by the cells. In one particular embodiment, the engineered yeast cells have been modified by providing a mutation in a gene encoding a membrane protein, so as to facilitate the uptake of proteins. Most particularly, the engineered cells of the present invention comprise a mutation in the gene encoding the erg6 protein. Other proteins which have been described to increase sensitivity to certain compounds in yeast strains are the transcription factors pdr1 and pdr3 (http://dtp.nci.nih.gov/yacds) and proteins involved in multi-drug resistance (e.g. Snq2, Yor1, Pdr11, Pdr10, Pdr15).

According to particular embodiments of the present invention, the engineered cells further comprise, in addition to the constructs described herein, a DNA sequence, the expression of which is suspected to affect protein misfolding. According to this aspect, the engineered cells of the invention are used to evaluate the effect of one or more compounds on protein misfolding, whereby these compounds are introduced into the cell or generated within the cell as the result of the introduction of a foreign DNA into the cell. Typically, a cDNA library of sequences is introduced into a multitude of cells so as to identify a cDNA sequence which, upon expression in the cell, is capable of affecting protein misfolding. This allows the monitoring of the effect of the protein encoded by the introduced cDNA on protein misfolding. Suitable expression vectors are well known in the art and will depend on the nature of the cells and the expression library used. For the yeast engineered cell line, the cDNA library can be of heterologous or of autologous origin e.g. a mammalian expression library or a yeast expression library, respectively.

The transgene(s) (reporter genes as well as optionally the one or more genes encoding one or more proteins prone to detrimental protein aggregation and/or the cDNA libraries) introduced into the engineered cells of the present invention may be introduced by any suitable transfection technique including electroporation, calcium phosphate precipitation, lipofection or other methods known to those skilled in the art. According to a particular embodiment, the engineered yeast cells comprise the one or more reporter genes stably integrated in their genome. The one or more transgenes encoding one or more proteins prone to misfolding can be present in the cells as plasmids. Typically, where genes encoding counterselection markers are used, these are provided in a plasmid together with the one or more genes encoding one or more proteins prone to misfolding.

Another aspect of the invention provides for the use of the cells of the present invention to monitor protein misfolding and to identify factors affecting protein misfolding. The cells of the present invention serve as a model for the processes occurring in diseases associated with aberrant protein folding.

Screening methods are provided which allow the identification of compounds with potential therapeutic benefits in the treatment and/or prevention of diseases involving detrimental protein aggregation and/or misfolding. Such diseases include, but are not limited to α-synucleinopathies (including Parkinson's disease, diffuse Lewy body dementia (also known as Lewy body disease), multiple system atrophy, Shy-Drager syndrome, neurologic orthostatic hypotension, Shy-McGee-Drager syndrome, and Parkinson's plus syndrome), tauopathies (including Alzheimer's disease, Pick's disease, corticobasal degeneration, lobar atrophy, progressive supranuclear palsy and frontotemporal dementia and Parkinsonism linked to chromosome 17 (FTDP-17)), amyloidoses (e.g. Alzheimer's disease, primary systemic amyloidosis, secondary systemic amyloidosis, familial amyloidotic polyneuropathy I, familial amyloidotic polyneuropathy II, senile systemic amyloidosis, hereditary cerebral amyloid angiopathy, haemodialysis-related amyloidosis, Finnish hereditary amyloidosis, type 2 diabetes, medullary carcinoma of the thyroid, atrial amyloidosis, lysozyme amyloidosis, insulin-related amyloidosis, fibrinogen α-chain amyloidosis, familial Mediterranean fever, Muckle-Wells' syndrome, isolated atrial amyloidosis, aortic medial amyloidosis, Down syndrome-related amyloidosis, congophilic angiopathy, Appalachian type amyloidosis, and prion diseases), prion diseases (including transmissible spongiform encephalopathies, Creutzfeldt-Jakob disease, fatal familia insomnia, Gerstmann-Straüssler Scheinker disease, mad cow disease or bovine spongiform encephalopathy, scrapie and kuru), serpinopathies (including α1-antitrypsin deficiency, familial encephalopathy with neuroserpin inclusion bodies and thrombosis), amyotrophic lateral sclerosis, Huntington's disease, spinocerebellar ataxia (type 1, type 2, type 3 or Machado-Joseph disease, type 6, type 7 and type 17), spinobulbar muscular atrophy (also known as Kennedy's Disease), dentatorubro-pallidoluysian atrophy or Haw River syndrome, inclusion body myositis, chronic lung diseases involving surfactant protein C, hypercholesterolaemia, cystic fibrosis, phenylketonuria, maple syrup urine disease or branched-chain ketonuria, Marfan syndrome, osteogenesis imperfecta, sickle cell anaemia, Tay-Sachs disease, scurvy, retinal dystrophies, retinitis pigmentosa, cataracts, familial corneal amyloidosis, inherited corneal dystrophies, laticce corneal dystrophies, pseudoexfoliation syndrome, heredo-oto-ophthalmo encephalopathy, cancer (involving misfolding of tumor suppressor, e.g. p53), oculopharyngeal muscular dystrophy, dystrophia myotonica, Friedreich's ataxia, hypotrichosis simplex of the scalp, cutaneous lichen amyloidosis, fragile X syndrome, fragile XE mental retardation, chronic liver diseases, atherosclerosis, DFNA9, corticobasal degeneration, emphysema, amyotrophic lateral sclerosis/parkinsonism dementia complex, Hirschprung disease, neurofibromatosis type 2, demyelinating peripheral neuropathies, Charcot-Marie-Tooth-like diseases, short-chain acyl-CoA dehydrogenase deficiency, idiopathic pulmonary fibrosis, argyrophilic grain disease, diffuse neurofibrillary tangles with calcification, frontotemporal dementia/parkinsonism linked to chromosome 17, Hallervorden-Spatz disease, Niemann-Pick disease type C, subacute sclerosing panencephalitis, haemolytic anemia, Wilson's disease, aging pituitary disorder, long QT syndrome or oculocutaneous albinism.

In the list provided above, the basis of the diseases may be genetic, idiopathic, sporadic or infectious, and all forms of these diseases are meant to be incorporated.

Cells as disclosed herein serve as a model for the processes occurring in diseases associated with aberrant protein folding of amyloidogenic proteins. Non-limiting examples of diseases resulting from misfolding and subsequent aggregation of amyloidogenic proteins include: α-synucleinopathies (including Parkinson's disease, diffuse Lewy body dementia (also known as Lewy body disease), multiple system atrophy, Shy-Drager syndrome, neurologic orthostatic hypotension, Shy-McGee-Drager syndrome, and Parkinson's plus syndrome), tauopathies (including Alzheimer's disease, Pick's disease, lobar atrophy, corticobasal degeneration, progressive supranuclear palsy and frontotemporal dementia and Parkinsonism linked to chromosome 17 (FTDP-17)), amyloidoses (e.g. Alzheimer's disease, primary systemic amyloidosis, secondary systemic amyloidosis, familial amyloidotic polyneuropathy I, familial amyloidotic polyneuropathy II, senile systemic amyloidosis, hereditary cerebral amyloid angiopathy, haemodialysis-related amyloidosis, Finnish hereditary amyloidosis, type 2 diabetes, medullary carcinoma of the thyroid, atrial amyloidosis, lysozyme amyloidosis, insulin-related amyloidosis, fibrinogen α-chain amyloidosis, familial Mediterranean fever, Muckle-Wells' syndrome, isolated atrial amyloidosis, aortic medial amyloidosis, Down syndrome-related amyloidosis, congophilic angiopathy, Appalachian type amyloidosis, and prion diseases), prion diseases (including transmissible spongiform encephalopathies, Creutzfeldt-Jakob disease, fatal familia insomnia, Gerstmann-Straüssler Scheinker disease, mad cow disease or bovine spongiform encephalopathy, scrapie and kuru), amyotrophic lateral sclerosis, Huntington's disease, spinocerebellar ataxia (type 1, type 2, type 3 or Machado-Joseph disease, type 6, type 7 and type 17), spinobulbar muscular atrophy (also known as Kennedy's Disease), dentatorubro-pallidoluysian atrophy or Haw River syndrome, inclusion body myositis, chronic lung diseases involving surfactant protein C, retinal dystrophies, retinitis pigmentosa, cataracts, familial corneal amyloidosis, inherited corneal dystrophies, laticce corneal dystrophies, pseudoexfoliation syndrome, heredo-oto-ophthalmo encephalopathy, cancer (involving misfolding of tumor suppressor, e.g. p53), hypotrichosis simplex of the scalp, cutaneous lichen amyloidosis, corticobasal degeneration, amyotrophic lateral sderosis/parkinsonism dementia complex, argyrophilic grain disease, frontotemporal dementia/parkinsonism linked to chromosome 17, Niemann-Pick disease type C or aging pituitary disorder. The basis for several of the listed diseases may be genetic, idiopathic, sporadic or infectious, and all forms of these diseases are meant to be incorporated.

Particular embodiments of this aspect of the invention provides screening methods to identify compounds or conditions capable of affecting protein misfolding and/or aggregation. Most particularly, the present invention provides the use of engineered cells according to embodiments of the present invention in the screening of compounds or conditions that affect (or have an effect on) protein misfolding and/or aggregation The effect of the compounds or conditions can be positive or stimulatory (i.e. increasing protein misfolding), negative or inhibitory (i.e. reducing protein misfolding) or neutral (no change in the overall misfolded proteins). The effect can be either direct (e.g. by binding one or more factors involved in the process of protein misfolding), or indirect (e.g. by stimulating the expression of a factor which itself affects protein misfolding). Typically, an increase or decrease of the reporter gene activity as determined in the methods of the present invention is indicative of a change in misfolding.

In specific embodiments of the present invention, methods of identifying compounds or conditions that directly or indirectly affect protein misfolding and eventual aggregation are provided, which methods comprise contacting the engineered cells of the invention with a compound of interest and monitoring the effect of the compound on protein misfolding and/or aggregation based on the signal of the reporter gene in the engineered cells. The term "contacting" in this context encompasses both administering the compound to the cells and providing cells wherein the compound (RNA, protein) is expressed.

In particular embodiments, the methods of the invention comprise adding a compound to the engineered cells described herein, and monitoring the effect of the compound on protein misfolding and/or aggregation based on the signal of the reporter gene in the engineered cells.

According to one embodiment, the methods of the present invention comprise the steps of:
a) providing engineered yeast cells, described herein comprising:
   - one or more reporter gene(s) under transcriptional control of a promoter of a heat shock protein, and
   - one or more gene(s) encoding one or more protein(s) prone to protein misfolding,
   whereby the engineered cells are characterized by spontaneous or induced misfolding of the one or more protein(s) prone to misfolding;
b) contacting the engineered yeast cells with the compound or condition; and
c) detecting the expression of the reporter gene so as to determine whether or not the compound or condition has affected aggregation and/or misfolding of the one or more proteins prone to misfolding.

Typically, a plurality of compounds is screened simultaneously or sequentially, whereby in parallel/consecutive experiments each compound is contacted with the engineered cells of the invention and the effect thereof on the cells (e.g. compared to a control) is determined.

According to the present invention, spontaneous or induced misfolding of the protein prone to misfolding and the effect thereon of compounds added to or introduced into the cell or of conditions to which the cell is subjected, can be detected in a sensitive way. While the effect of misfolding on survival or growth of the cells may not be directly detectable or quantitatively representative, misfolding of the one or more proteins prone to misfolding will affect the expression of the reporter gene, which itself can be directly detected in a sensitive and quantitative way. In the same way, the effect of contacting the cells with one or more compounds which potentially affect protein misfolding and/or aggregation can be monitored based on reporter gene expression. Thus, an advantage of the methods and assays of the present invention, is that they allow a more sensitive and quantitative monitoring of protein aggregation and/or misfolding, than methods based on the detection of aggregation itself.

An additional advantage is that the procedure can be done in yeast cells, ensuring a physiologically more relevant cellular context for expression of human proteins compared to prokaryotic model systems. This is an important aspect since folding and misfolding of proteins is at least in part modulated by eukaryote-specific post-translational protein modifications and/or by the cellular environment (e.g. oxidative stress generated by mitochondria).

A further additional advantage is that the methods of the invention allow the use of native coding sequences of proteins prone to misfolding; it is not necessary to fuse or link them with additional coding sequences (e.g. encoding for subcellular targeting signals or protein tags such as GFP, HA) for detection, or with membrane anchoring sequences to ensure aggregation as described for the screening systems of the prior art. However, if desired, such chimeric proteins can be used in the context of the invention.

Typically, the methods of the present invention involve the culturing, growing or suspending of the cell(s) in an appropriate medium, suitable for the growth of a non-engineered cell of the same species. Suitable cultivation conditions for the cell lines of the present invention are known in the art.

In one embodiment of the invention, the reporter gene encodes a protein, the expression of which is detectable by subjecting the cells to particular cultivation conditions. According to this embodiment, the methods further provide the step of cultivating the cells under appropriate conditions or appropriate medium to allow the detection of the expression of the reporter gene. For instance, in a particular embodiment, the detection step comprises plating yeast cells on a medium so as to allow identification of the colonies in which the reporter gene is expressed. Typically, the appropriate medium is a medium which has been supplemented with one or more substances that allow the detection of the expression of the reporter gene product or from which one or more substances are removed. Most particularly, the reporter gene is a gene encoding a protein required in the synthesis of an essential amino acid, and the methods of the present invention comprise the step of cultivating the engineered cells of the invention in a selection medium not comprising the relevant essential amino acid and identifying the cells showing enhanced growth and/or differentiation in the selection medium. According to an alternative embodiment, the reporter gene encodes a protein conferring a particular resistance, such as a stress, pathogen or antibiotic resistance, and the methods of the invention comprise the step of subjecting the cell to the corresponding stress, pathogen or antibiotic, and identifying the cells showing enhanced growth and/or differentiation in the selection medium. Depending on the reporter gene used, the selection medium can further comprise additional reporter gene modulating compounds (described above) which enhance the selectivity of the reporter gene.

Typically in the methods of the present invention the contacting of the engineered yeast cells with one or more compounds is ensured by adding the relevant compound(s) to the medium of the cells. For many compounds, this will ensure uptake of the compounds by the cells. Additionally or alternatively selective uptake of the compounds can be envisaged e.g. by targeting or by providing the compounds in a formulation which increases uptake by the cells. Further embodiments of the invention envisage (chemical or genetic) modulation of the cells so as to increase the uptake of the compounds by the cells (see below) or the generation of the compounds within the cells (e.g. cDNA libraries, also discussed more in detail below).

According to one embodiment, the methods of the invention make use of engineered cells comprising a reporter gene and one or more genes encoding one or more proteins prone to misfolding or aggregation, characterized by spontaneous misfolding of the one or more proteins prone to misfolding. According to this embodiment, the step of providing the engineered cells of the invention characterized by spontaneous or inducible misfolding includes the step of cultivating engineered cells comprising at least one reporter gene and at least one gene encoding at least one protein prone to (protein) misfolding under conditions which allow spontaneous or induced mutations to occur, so as to ensure the generation of engineered cells in which spontaneous misfolding of the protein prone to misfolding occurs. In such cells, reporter gene expression is induced or enhanced, and these cells can be identified and selected based on the expression of the reporter gene.

According to an alternative embodiment, the methods of the invention make use of an engineered cell comprising a reporter gene and one or more genes encoding one or more proteins prone to misfolding or aggregation, characterized by inducible misfolding of the one or more proteins prone to misfolding, such as those described above. In this embodiment, the methods of the invention further comprise the step of contacting the cells with an external factor capable of inducing misfolding of the one or more proteins prone to misfolding. This step can be ensured prior to or simultaneously with the step of the methods of the invention whereby the engineered cells are contacted with the test compounds. Where the test compounds are expressed within the cell (e.g. cDNA libraries), misfolding of the protein is induced upon expression of the compound(s) in the cell.

According to one embodiment, the methods of the present invention further comprise a negative selection or counter selection step. This is of interest to ensure that the observed effect on the reporter gene is linked to the one or more proteins prone to misfolding. Typically, where the one or more proteins prone to misfolding are encoded by a transgene, the transgene is linked to a marker gene encoding a counterselection marker and the counterselection step encompasses adding a compound to the medium which, upon expression of the counter-selection marker by the cells, is converted to a product which is toxic to the cells. Optionally, the negative selection step is performed as part of a control step, whereby removal of one or more of the transgenes is ensured.

Where the protein prone to misfolding is an endogenous protein, counterselection can be done by knockout of the endogenous gene encoding this protein, or by inhibiting expression of its gene product, e.g. by RNAi.

The screening methods of the present invention are suitable for the screening of any type of compound. Typically, the compound are compounds such as, but not limited to, chemical compounds (including small molecules e.g. molecules less than 30 kDa), proteins, peptides, antibodies or fragments thereof. The methods of the present invention are suitable for high-throughput screening and thus can be used in the screening of chemical libraries or parts thereof, peptide libraries or parts thereof, etc.

According to particular embodiments, screening methods provided in the context of the present invention involve comparing the expression of the reporter gene upon addition of the test-compound with a control, such as a positive or negative control. A negative control can be a culture of the same engineered cells of the invention to which no compound has been added, a culture of cells to which a blank (compound known not to affect protein aggregation) has been added, or the culture of cells prior to the addition of the compound. A positive control can be a culture of the same engineered cells to which a compound has been added known to (positively or negatively) affect protein misfolding and subsequent aggregation.

Thus, according to these embodiments, screening methods additionally comprise the step of comparing the effect of the test compound with the effect of a positive and/or negative control.

The detection step in methods of the present invention is determined by the nature of the reporter gene. Where the reporter gene directly or indirectly (e.g. as a result of reacting with a substrate present in the medium) ensures the generation of a product which can be optically detected, typical detection methods involve the use of detectors which are spectrophotometric devices, UV detectors, fluorescent detection devices etc. Additionally or alternatively, where the expression of the reporter gene directly or indirectly affects the growth of the cells, the effect of the test compounds on expression of the reporter gene can be determined based on growth or differentiation of the cells. Typically, in order to determine the growth of a cell culture, the density of the culture at OD₅₉₅ is determined in a standard micro plate reader.

According to a further aspect, methods are provided to screen cDNA collections for cDNA's encoding proteins or peptides that directly or indirectly affect protein misfolding and/or aggregation. According to one embodiment, the methods of the present invention comprise the steps of:
a) providing a cell line or cell population comprising
   - at least one reporter gene under transcriptional control of a promoter of a heat shock protein, and
   - one or more gene(s) encoding one or more protein(s) prone to misfolding,
   whereby the engineered cells are characterized by spontaneous or inducible misfolding of the one or more protein(s) prone to misfolding;
b) transforming the cell line or cell population with an expression cDNA library so as to generate individual cells expressing one or more cDNAs of the cDNA library; and
c) detecting the expression of the reporter gene in the cells so as to determine the effect of the cDNA introduced on protein misfolding in the cell.

Besides the fact that in this aspect the compounds to be screened are generated inside the cell rather than added to the cell, methods envisaged according to this aspect of the present invention are similar to those described above. Again, a particular embodiment of this aspect of the invention involves the use of cells characterized by spontaneous misfolding of the one or more proteins prone to misfolding. More particularly, the yeast cells used in this aspect are characterized by spontaneous misfolding and are obtainable by cultivating engineered yeast cells comprising at least one reporter gene under transcriptional control of a promoter of a heat shock protein, and one or more gene(s) encoding one or more protein(s) prone to misfolding and selecting yeast colonies with elevated reporter gene expression.

According to particular embodiments, the effect of the introduction of the cDNA on expression of the reporter gene is compared with a positive or negative control, e.g. a cell culture that was not transformed with cDNA, a cell culture that was transformed with an empty vector, the cell culture prior to the transformation with cDNA, a cell culture transformed with cDNA encoding proteins or peptides with a known effect on protein aggregation, or a cell culture transformed with cDNA encoding structurally or functionally related proteins or peptides.

Thus, according to this embodiment, the screening methods further comprise the step of providing a positive and/or negative control and the detection step (c) comprises comparing the expression of the reporter genes in the engineered cells comprising a cDNA of the cDNA library to the expression of the reporter gene in the positive and/or negative controls.

The methods of the invention can be used as an assay, an automated assay or a high throughput screening assay. Since yeast combines ease of manipulation with the possibility of cost-effective screening.

It is envisaged that agents, compounds, proteins and/or peptides identified using the methods of the invention might offer valuable therapeutic leads for treatment of protein conformational diseases. Mixing compounds identified using these methods, or derivatives or homologues thereof, with a pharmaceutically acceptable carrier is a way of obtaining new pharmaceutical compositions.

The invention is further illustrated by following examples. It is to be appreciated by those skilled in the art that these examples are not meant as limiting, but intend to further clarify the present invention.

### EXAMPLES

### Example 1 - Construction of yeast strains bearing a HIS3 reporter gene under control of the Hsf1-controlled SSA3 promoter (pSSA3-HIS3 strains)

A DNA fragment was generated by PCR using primers **SSA3-F:** CTT GTA TGT CAA TGT TTG TCA CTA AAC GGA TAG AAT AGG TAC TAA ACG CTA CAA AGA AAA ATG ACA GAG CAG AAA GCC CTA GTA AAG CGT ATT ACA AAT G (SEQ ID NO:1) and **SSA3-R:** CGC CAT CGT ATA AAA GGT TAA ACA TAA AAA GTA GCT AAA TAG AAC ACT ATA GAA GAA TAA CTA CAT AAG AAC ACC TTT GGT GGA GGG AAC ATC G (SEQ ID NO: 2) and a plasmid-derived yeast *HIS3* gene as template. This PCR fragment contained the *HIS3* sequence flanked by the immediate upstream and downstream flanking regions of the yeast *SSA3* coding region. This DNA fragment was subsequently transformed to yeast strain W303-1 A (Thomas, B.J. and Rothstein, R. (1989) Cell 56, 619-630)) resulting in an exchange of the *SSA3* coding region for the *HIS3* coding region by means of homologous recombination on *SSA3* flanking regions. Transformants were selected on solid SC-HIS medium and individual colonies were checked by PCR for correct genomic integration of the reporter gene.

### General yeast media and culture conditions

Yeast cells were cultured in microtiter plates (150µL/well) in standard yeast media as described elsewhere (M. Rose, F. Winston and P. Hieter, Methods in yeast genetics, a laboratory course manual., Cold Spring Harbor Laboratory Press, Cold Spring harbor, N. Y. (1990)). In the present examples, the reporter gene used encodes an compound required in the synthesis of an essential amino acid. Accordingly, synthetic complete (SC) medium supplemented with adenine, uracil and amino acids, but lacking the corresponding essential amino acid was used to enforce selection. In addition, 3AT (3-amino-1,2,4-triazole), an inhibitor of the His3 enzyme, was added to the growth medium. 3AT is added to SC-HIS medium (3AT selection medium) at concentrations in which histidin biosynthesis (as a function of *pSSA3-HIS3* expression) is limiting for growth. Growth was monitored by measuring optical density at 595 nm using a standard microtiter plate reader.

### Example 2 - Isolation of pSSA3-HIS3 strains in which reporter activation is triggered specifically by proteins prone to protein misfolding

Noxious protein aggregation is a multi-step process starting with the formation of alternative non-native conformations (misconformers), most often possessing the intrinsic property to self-polymerise into higher order oligomers and ultimately to relatively large insoluble aggregates (Figure 1, upper part). The formation of non-native protein conformations triggers activation of transcription factors such as Hsf1. In the generated *pSSA3-HIS3* strain, the Hsf1-controlled *SSA3* promoter is fused to a reporter gene *HIS3* encoding the His3 enzyme required for the biosynthesis of the essential amino acid histidin (Figure 1, lower part). Hsf1-dependent activation by protein misfolding results in increased expression of *HIS3,* therefore allowing faster growth on histidin-limited growth medium. 3AT is an inhibitor of His3 enzyme and is used to inhibit background expression of *HIS3* (i.e. basal level of expression in the absence of misfolding and/or aggregation) in order to make histidin biosynthesis limited for optimal growth.

*pSSA3-HIS3* strains were transformed with an expression plasmid bearing cDNA encoding a particular protein prone to protein misfolding (e.g. α-synuclein). The presence of aggregation-prone proteins is not sufficient (at least in wild type yeast strains) for activation of Hsf1-mediated *HIS3* expression and consequently does not allow growth on 3AT selection medium significantly better than vector transformed control strains (fig. 2, step 1; visualised by the thin arrow).

In order to obtain strains with strong protein-misfolding directed Hsf1-activation a screen was undertaken to identify *pSSA3-HIS3* transformants presumably bearing (a) genomic DNA mutation(s) that allow growth on 3AT selection medium (by sufficient activation of the reporter gene). To this end, *pSSA3-HIS3* transformants were grown in liquid medium and plated on solid SC-HIS medium with 40 mM 3AT in order to identify spontaneous 3AT resistant mutants with strong expression of *HIS3.* In figure 2 (step 2), such spontaneous genomic mutants are depicted by a star. Improved growth on 3AT selection medium is visualised by a fat arrow. Growth depends on specific activation of Hsf1 by the protein prone to protein misfolding involved, and some genomic mutations may enhance this activation. For instance, mutations that slow down clearance of misfolded proteins would lead to higher levels of misfolded proteins and thereby to a stronger activation of the *SSA3* promoter.

To reassure that reporter activation (and thus growth) is specifically dependent on misfolding and/or aggregation of the protein prone to protein misfolding, the identified 3AT resistant mutant strains were evaluated individually. To this end, the plasmid bearing a cDNA encoding a particular aggregation-prone protein is removed by 5FOA counterselection of the plasmid borne *URA3.* The strains are subsequently re-transformed with a corresponding empty vector (fig. 2, step 3).

Growth on 3AT selection medium of individual 3AT resistant mutants (identified in step 2) was compared with the corresponding strains that had undergone 5FOA counterselection (of the expression plasmid) and were re-transformed with corresponding empty vector (step 3 transformants). The *pSSA3-HIS3* mutants retransformed with empty vector that grew significantly slower on 3AT selection medium (as visualised by the thin arrow in fig. 2, step 3) compared to the corresponding mutants producing the protein prone to protein misfolding, are strains in which the Hsf1-driven *pSSA3-HIS3* reporter is activated specifically by the aggregation-prone protein involved.

Following this approach p*SSA3-HIS3* strains with plasmids rUd-TAU (example 3) or rULd-SYN (example 4) were identified that displayed better growth on 3AT selective medium relative to corresponding empty vector (rUd or rULd, respectively) transformants. In these strains Hsf1-driven p*SSA3-HIS3* reporter is specifically activated by respectively, tau and α-synuclein.

### Example 3 - Expression of a gene encoding human tau activates an Hsf1-regulated promoter in yeast

### Yeast expression plasmids

Plasmids rUd (empty vector control) and rUd-TAU (to express human tau in yeast) were used. Construction of plasmids rUd and rUd-TAU: plasmid pJW212T (Griffioen et al. (2006) BBA 1762, 312-318) was cut with *Sbf*I/*Nru*I, treated with Klenow to blunt end overhangs and religated. This resulted in plasmid rUd. cDNA encoding human 2N/4R tau was subsequently inserted in rUd (*Eco*RI/*Xh*oI) to create plasmid rUd-TAU.

### Growth assay to detect tau-triggered Hsf1 activation in pSSA3-HIS3 transformant H9

Following the procedures as outlined above in examples 1 and 2 a *pSSA3-HIS3* strain (H9) was isolated with tau-responsive *HIS3* expression. H9 transformed with rUd (vector) or with rUd-TAU were grown to stationary phase in SC-URA medium. From this culture cells were inoculated in SC-HIS medium supplemented with 3 mM 3AT at a cell density of approximately 1,8*10⁷ cells/mL and grown 24 hours at 30°C (200 rpm). From this pre-culture cells were inoculated (micro plates) in SC-HIS (assay medium) or SC medium (growth control) supplemented 3AT (usually 0,15 mM but higher concentrations can also be used) at a cell density of approximately 7,5*10⁵ cells/mL and grown at 30°C. OD₅₉₅ was determined using a standard micro plate reader.

Growth of H9 bearing rUd (fig. 3, open bars) and rUd-TAU (fig. 3, solid bars) was assessed on complete medium and 3AT-selection medium (0,5 mM 3AT). H9 expressing tau, but not rUd transformants, grew better on histidin-selection medium indicating that intracellular tau specifically activates expression of the *pSSA3-HIS3* reporter. In contrast to histidin-limited medium, in complete medium with abundant histidin available no major difference of growth was observed between the two transformants indicating that *HIS3* expression in this strain is limited for optimal growth on 3AT selection medium (fig. 3).

### Example 4 - Expression of a gene encoding human α-synuclein activates an Hsf1-regulated promoter in yeast

### Yeast expression plasmids

Plasmids rULd (empty vector control) and rULd-SYN (to express human α-synuclein in yeast) were used. Construction of plasmids rULd and rULd-SYN: a DNA fragment was generated by PCR using primers **LEU2-F:** GGA ATT CGG AGC TCT ATA TAT ATT TCA AGG ATA TAC CAT TCT AAT G (SEQ ID NO:3) and **LEU2-R** (SEQ ID NO:4): GGA ATT CGC CTG CAG GCA TCT CCA TGC AGT TGG ACG ATC GAT G and a plasmid-derived yeast *LEU2* gene as template. This DNA fragment was subcloned in pJW212T (*Sac*I/*Sbf*l) resulting in plasmid rLd. rLd-SYN was created by subcloning *(Nco*I/*Xho*I) cDNA encoding α-synuclein in rLd. Subsequently, an *AatI*I-*Nae*I from pRS306 (Sikorski, R.S., and Hieter, P. (1989) Genetics 122, 19-27) containing yeast *URA3* was subcloned *Aatl*I*-NgoM*IV in rLd and rLd-SYNwt resulting in plasmids rULd and rULd-SYN.

### Growth assay to detect α-synuclein-triggered Hsf1 activation in pSSA3-HIS3 transformant B11

Following the procedures as outlined above in examples 1 and 2 a p*SSA3-HIS3* strain (B11) was isolated with α-synuclein-responsive *HIS3* expression. B11 transformed with rULd (vector) or with rULd-SYN (α-synuclein) were grown to stationary phase in SC-LEU medium. From this culture cells were inoculated in SC-HIS medium supplemented with 3 mM 3AT at a cell density of approximately 5*10⁶ cells/mL and grown overnight at 30°C (200 rpm). From this pre-culture cells were inoculated (micro plates) in SC-HIS (assay medium) or SC medium (growth control) supplemented 3AT (usually 4 mM but lower concentrations can also be used) at a cell density of approximately 7,5*10⁵ cells/mL and grown at 30°C. OD₅₉₅ was determined using a standard micro plate reader.

Growth of B11 bearing rULd (fig. 4, open bars) and rULd-α-synuclein (fig. 4, solid bars) was assessed on complete medium and 3AT-selection medium (2 mM 3AT). B11 expressing α-synuclein, but not rULd transformants, grew better on histidin-selection medium indicating that intracellular α-synuclein specifically activates expression of the *pSSA3-HIS3* reporter. In contrast to histidin-limited medium, in complete medium with abundant histidin available, no major difference of growth was observed between the two transformants indicating that *HIS3* expression in this strain is limited for optimal growth on 3AT-selection medium (fig. 4).

### Example 5 - Assessment of the quality of the assay of the invention

Robustness and reproducibility of the assay described in the present invention was determined using the Z'-factor (Zhang, J. H., Chung, T. D., and Oldenburg, K. R. (1999) J Biomol Screen 4, 67-73). An assay with a Z'-factor of higher than 0,5 is considered to be suitable for high-throughput screening of, for instance, compound libraries. Z'-factors were calculated using OD₅₉₅ measurements of histidin-limited *pSSA3-HIS3* strains transformed with empty vector or the corresponding plasmid with cDNA encoding an amyloidogenic proteins (TAU or α-synuclein).

The Z'-factor indicated in Figures 3 and 4 demonstrates excellent assay quality of both the tau-based and the α-synuclein based assays, suitable for high-throughput screening.

## Claims

1. A method for determining whether a compound or a plurality of compounds is/are capable of directly or indirectly affecting protein misfolding, said method comprising the steps of:
a) providing a culture of engineered yeast cells, comprising one or more reporter gene(s) under transcriptional control of a promoter of a heat shock protein, and further comprising one or more gene(s) encoding one or more protein(s) prone to misfolding, wherein the engineered yeast cells are **characterized by** spontaneous or inducible misfolding of said protein(s) prone to misfolding; and
b) contacting the cells with said compound or said plurality of compounds and detecting the activity of said reporter gene product in said cells to determine the effect of said compound or said plurality of compounds on protein misfolding in said engineered cells.
wherein the protein prone to misfolding is selected from the group consisting of serpins, poly(A) binding protein 2, low-density lipoprotein receptor, apolipoprotein B100, cochlin, RET, myelin protein 22/0, short-chain acyl CoA dehydrogenase, cystic fibrosis transmembrane conductance regulator, HERG, phenylalanine hydroxylase, fibrillin, procollagen, collagen, haemoglobin, ATPase ATP7B, β-hexosaminidase tyrosinase, amyloid precursor protein, tau, α-synuclein, huntingtin, ADan peptide, ABri peptide, amyloid β, prion protein, ataxins, superoxide dismutase, cystatin C, atrophin 1, androgen receptor, TATA box-binding protein, transthyretin, serum amyloid A, Ig light chains, β2-microglobulin, apolipoprotein A-1 , gelsolin, pro- islet amyloid polypeptide, procalcitonin, lysozyme, insulin, fibrinogen α-chain, atrial natriuretic factor, surfactant protein C, rhodopsin, γ-crystallins, lactoferrin, βig-h3, kerato-epithelin, corneodesmosin, p53, lactadherin and prolactin.

2. The method according to claim 1, which is a method for screening a plurality of compounds capable of directly or indirectly affecting protein misfolding,
wherein said compounds are cDNAs or the expression products thereof, comprising the steps of:
a) providing a culture of engineered yeast cells comprising one or more reporter gene(s) under transcriptional control of a promoter of a heat shock protein, and one or more gene(s) encoding one or more protein(s) prone to misfolding, the engineered yeast cell being **characterized by** spontaneous or inducible misfolding of said protein(s) prone to misfolding;
b) transforming the culture of engineered cells with an expression cDNA library so as to obtain a culture of transformed cells each comprising one or more cDNAs; and,
c) detecting the activity of the reporter gene in the transformed cells of the culture to determine the effect of the expression product of said one or more cDNAs on misfolding in said transformed cells.

3. The method according to claim 1 or 2 wherein said engineered yeast cells are **characterized by** spontaneous misfolding of the one or more protein(s) prone to misfolding and step (a) comprises:
- cultivating engineered yeast cells comprising one or more reporter gene(s) under transcriptional control of a promoter of a heat shock protein and one or more gene(s) encoding one or more protein(s) prone to misfolding, under conditions which allow and/or induce mutations of the genome of the engineered yeast cells resulting in spontaneous misfolding of the protein(s) prone to misfolding, and
- selecting a cell **characterized by** spontaneous misfolding based on increased expression of the one or more reporter gene(s) compared to the engineered yeast cells before cultivation.

4. The method according to any one of claims 1 to 3, wherein said cells are **characterized by** inducible misfolding of the protein(s) prone to misfolding and the method further comprises, prior to step (b) the step of contacting the engineered yeast cells with one or more external factors capable of selectively inducing misfolding of the one or more proteins prone to misfolding.

5. The method according to claim 4, wherein at least one of said protein(s) prone to misfolding is a-synuclein and the external factor capable of selectively inducing misfolding of the one or more protein(s) prone to misfolding is selected from the group consisting of paraquat, rotenone and MPTP.

6. The method of any one of claims 1 to 5, wherein at least one of said one or more protein(s) prone to protein misfolding is an amyloidogenic protein selected from the group consisting , amyloid precursor protein, tau, α-synuclein, huntingtin, ADan peptide, ABri peptide, amyloid β, prion protein, ataxins, superoxide dismutase, cystatin C, atrophin 1, androgen receptor, TATA box-binding protein, transthyretin, serum amyloid A, Ig light chains, β2-microglobulin, apolipoprotein A-1 , gelsolin, pro- islet amyloid polypeptide, procalcitonin, lysozyme, insulin, fibrinogen α-chain, atrial natriuretic factor, surfactant protein C, rhodopsin, γ-crystallins, lactoferrin, βig-h3, kerato-epithelin, corneodesmosin, p53, lactadherin and prolactin.

7. The method according to any of claims 1 to 6, wherein at least one of said one or more reporter genes encodes a protein with an activity that positively or negatively affects growth and/or proliferation of said engineered yeast cells.

8. A yeast cell , having a transgene present as an extrachromosomal element or integrated into its genomic DNA, said cell comprising:
a) one or more reporter gene(s) under transcriptional control of a promoter of a heat shock protein; and
b) one or more transgene(s) encoding one or more protein(s) prone to misfolding, wherein the protein prone to misfolding is selected from the group consisting of serpins, poly(A) binding protein 2, low-density lipoprotein receptor, apolipoprotein B100, cochlin, RET, myelin protein 22/0, short-chain acyl CoA dehydrogenase, cystic fibrosis transmembrane conductance regulator, HERG, phenylalanine hydroxylase, fibrillin, procollagen, collagen, haemoglobin, ATPase ATP7B, β-hexosaminidase tyrosinase, amyloid precursor protein, tau, α-synuclein, huntingtin, ADan peptide, ABri peptide, amyloid β, prion protein, ataxins, superoxide dismutase, cystatin C, atrophin 1, androgen receptor, TATA box-binding protein, transthyretin, serum amyloid A, Ig light chains, β2-microglobulin, apolipoprotein A-1 , gelsolin, pro- islet amyloid polypeptide, procalcitonin, lysozyme, insulin, fibrinogen α-chain, atrial natriuretic factor, surfactant protein C, rhodopsin, γ-crystallins, lactoferrin, βig-h3, kerato-epithelin, corneodesmosin, p53, lactadherin and prolactin

9. The cell according to claim 8, wherein at least one of said one or more proteins prone to misfolding is an amyloidogenic protein selected from the group consisting of , amyloid precursor protein, tau, α-synuclein, huntingtin, ADan peptide, ABri peptide, amyloid β, prion protein, ataxins, superoxide dismutase, cystatin C, atrophin 1, androgen receptor, TATA box-binding protein, transthyretin, serum amyloid A, Ig light chains, β2-microglobulin, apolipoprotein A-1 , gelsolin, pro-islet amyloid polypeptide, procalcitonin, lysozyme, insulin, fibrinogen α-chain, atrial natriuretic factor, surfactant protein C, rhodopsin, γ-crystallins, lactoferrin, βig-h3, kerato-epithelin, corneodesmosin, p53, lactadherin and prolactin

10. A culture of yeast cells according to claim 8 or 9, **characterized in that** said cells comprise an expression vector comprising a cDNA.

11. A method to generate a yeast cell for monitoring protein misfolding, comprising the steps of:
a) providing engineered yeast cells comprising:
- one or more reporter gene(s) under transcriptional control of a promoter of a heat shock protein, and
- one or more genes encoding one or more proteins prone to misfolding,
b) cultivating said yeast cells under conditions which allow mutations of the genome of said yeast cells; and
c) selecting a cell wherein said one or more reporter gene(s) are expressed at higher levels, compared to the yeast cells prior to the cultivation
wherein prone to misfolding, wherein the protein prone to misfolding is selected from the group consisting of serpins, poly(A) binding protein 2, low-density lipoprotein receptor, apolipoprotein B100, cochlin, RET, myelin protein 22/0, short-chain acyl CoA dehydrogenase, cystic fibrosis transmembrane conductance regulator, HERG, phenylalanine hydroxylase, fibrillin, procollagen, collagen, haemoglobin, ATPase ATP7B, β-hexosaminidase tyrosinase, amyloid precursor protein, tau, α-synuclein, huntingtin, ADan peptide, ABri peptide, amyloid β, prion protein, ataxins, superoxide dismutase, cystatin C, atrophin 1, androgen receptor, TATA box-binding protein, transthyretin, serum amyloid A, Ig light chains, β2-microglobulin; apolipoprotein A-1 , gelsolin, pro-islet amyloid polypeptide, procalcitonin, lysozyme, insulin, fibrinogen α-chain, atrial natriuretic factor, surfactant protein C, rhodopsin, γ-crystallins, lactoferrin, βig-h3, kerato-epithelin, corneodesmosin, p53, lactadherin and prolactin

12. The method according to claim 11, wherein the cells are provided with a gene encoding a counterselection marker linked to said one or more genes encoding one or more proteins prone to misfolding and said method comprises the step of evaluating, by use of said counterselection marker, whether the reporter gene expression levels are dependent on the presence of said one or more genes encoding one or more proteins prone to misfolding.

13. Use of a cell according to claim 8 or 9, for the identification of compounds and/or conditions affecting protein misfolding, by detecting the activity of said reporter gene product in said cells upon application of said compounds and/or conditions.

## Patentansprüche

1. Verfahren zum Bestimmen, ob eine Verbindung oder mehrere Verbindungen imstande ist bzw. sind, direkt oder indirekt eine Proteinfehlfaltung zu beeinflussen, wobei das Verfahren die folgenden Schritte umfasst:
a) Bereitstellen einer Kultur manipulierter Hefezellen, umfassend ein oder mehrere Reportergen(e) unter transkriptionaler Kontrolle eines Promotors eines Hitzeschockproteins und des Weiteren umfassend ein oder mehrere Gen(e), das oder die für ein oder mehrere Protein(e) codiert bzw. codieren, das oder die zur Fehlfaltung neigt bzw. neigen, wobei die manipulierten Hefezellen durch spontane und induzierbare Fehlfaltung des Proteins oder der Proteine, das oder die zur Fehlfaltung neigt bzw. neigen, gekennzeichnet sind; und
b) In-Kontakt-Bringen der Zellen mit der Verbindung oder den mehreren Verbindungen und Nachweisen der Aktivität des Reportergenprodukts in den Zellen zur Bestimmung der Wirkung der Verbindung oder der mehreren Verbindungen auf eine Proteinfehlfaltung in den manipulierten Zellen,
wobei das Protein, das zur Fehlfaltung neigt, ausgewählt ist aus der Gruppe bestehend aus Serpinen, Poly(A)-Bindungsprotein 2, LDL ("low density lipoprotein" - Lipoprotein geringer Dichte)-Rezeptor, Apolipoprotein B100, Cochlin, RET, Myelinprotein 22/0, kurzkettiger Acyl-CoA-Dehydrogenase, CFTR (Cystic Fibrosis Transmembrane Conductance Regulator), HERG, Phenylalaninhydroxylase, Fibrillin, Prokollagen, Kollagen, Hämoglobin, ATPase ATP7B, β-Hexosaminidase-Tyrosinase, amyloidem Vorläuferprotein, tau, α-Synuklein, Huntingtin, ADan Peptid, ABri Peptid, amyloidem β, Prionprotein, Ataxinen, Superoxiddesmutase, Cystatin C, Atrophin 1, Androgenrezeptor, TATA-Box-Bindungsprotein, Transthyretin, Serum-Amyloid A, lg Leichtketten, β2-Mikroglobulin, Apolipoprotein A-1, Gelsolin, Pro-Islet-Amyloid-Polypeptid, Prokalzitonin, Lysozym, Insulin, Fibrinogen α-Kette, atrialem natriuretischen Faktor, Surfactant-Protein C, Rhodopsin, γ-Kristallinen, Laktoferrin, βig-h3, Kerato-Epithelin, Corneodesmosin, p53, Lactadherin und Prolaktin.

2. Verfahren nach Anspruch 1, das ein Verfahren für das Screening mehrerer Verbindungen ist, die imstande sind, direkt oder indirekt eine Proteinfehlfaltung zu beeinflussen,
wobei die Verbindungen c-DNAs oder deren Expressionsprodukte sind, umfassend die folgenden Schritte:
a) Bereitstellen einer Kultur manipulierter Hefezellen, umfassend ein oder mehrere Reportergen(e) unter transkriptionaler Kontrolle eines Promotors eines Hitzeschockproteins und ein oder mehrere Gen(e), das oder die für ein oder mehrere Protein(e) codiert bzw. codieren, das oder die zur Fehlfaltung neigt bzw. neigen, wobei die manipulierten Hefezellen durch spontane oder induzierbare Fehlfaltung des Proteins oder der Proteine, das oder die zur Fehlfaltung neigt bzw. neigen, gekennzeichnet sind;
b) Transformieren der Kultur manipulierter Zellen mit einer Expressions-cDNA-Bibliothek, um eine Kultur transformierter Zellen zu erhalten, die jeweils eine oder mehrere cDNAs umfassen; und
c) Nachweisen der Aktivität des Reportergens in den transformierten Zellen der Kultur zur Bestimmung der Wirkung des Expressionsprodukts der einen oder mehreren cDNAs auf die Fehlfaltung in den transformierten Zellen.

3. Verfahren nach Anspruch 1 oder 3, wobei die manipulierten Hefezellen durch spontane Fehlfaltung des einen Proteins oder der mehreren Proteine, das oder die zur Fehlfaltung neigt bzw. neigen, gekennzeichnet sind und Schritt (a) umfasst:
- Kultivieren manipulierter Hefezellen, umfassend ein oder mehrere Reportergen(e) unter transkriptionaler Kontrolle eines Promotors eines Hitzeschockproteins und ein oder mehrere Gen(e), das oder die für ein oder mehrere Protein(e) codiert bzw. codieren, das oder die zur Fehlfaltung neigt bzw. neigen, unter Bedingungen, die Mutationen des Genoms der manipulierten Hefezellen ermöglichen und/oder induzieren, was zu einer spontanen Fehlfaltung des Proteins oder der Proteine führt, das oder die zur einer Fehlfaltung neigt, bzw. neigen, und
- Selektieren einer Zelle, die durch eine spontane Fehlfaltung charakterisiert ist, auf der Basis einer erhöhten Expression des einen Reportergens oder der mehreren Reporteregene im Vergleich zu den manipulierten Hefezellen vor der Kultivierung.

4. Verfahren nach einem der Ansprüche 1 bis 3, wobei die Zellen durch eine induzierbare Fehlfaltung des Proteines oder der Proteine, das oder die zur Fehlfaltung neigt bzw. neigen, gekennzeichnet sind und das Verfahren des Weiteren vor Schritt (b) den Schritt des In-Kontakt-Bringens der manipulierten Hefezellen mit einem oder mehreren externen Faktor(en) umfasst, der oder die imstande ist bzw. sind, selektiv eine Fehlfaltung des einen Proteins oder der mehreren Proteine zu induzieren, das oder die zu einer Fehlfaltung neigt bzw. neigen.

5. Verfahren nach Anspruch 4, wobei mindestens eines von dem oder der Protein(e), das oder die zur Fehlfaltung neigt bzw. neigen, α-Synuklein ist und der externe Faktor, der zum selektiven Induzieren einer Fehlfaltung des einen Proteins oder der mehreren Proteine imstande ist, das oder die zu einer Fehlfaltung neigt bzw. neigen, ausgewählt ist aus der Gruppe bestehend aus Paraquat, Rotenon und MPTP.

6. Verfahren nach einem der Ansprüche 1 bis 5, wobei mindestens eines des einen Proteins oder der mehreren Proteine, das oder die zu einer Fehlfaltung neigt bzw. neigen, ein amyloidogenes Protein ist, das ausgewählt ist aus der Gruppe bestehend aus amyloidem Vorläuferprotein, tau, α-Synuklein, Huntingtin, ADan Peptid, ABri Peptid, amyloidem β, Prionprotein, Ataxinen, Superoxiddesmutase, Cystatin C, Atrophin 1, Androgenrezeptor, TATA-Box-Bindungsprotein, Transthyretin, Serum-Amyloid A, Ig Leichtketten, β2-Mikroglobulin, Apolipoprotein A-1, Gelsolin, Pro-Islet-Amyloid-Polypeptid, Prokalzitonin, Lysozym, Insulin, Fibrinogen α-Kette, atrialem natriuretischen Faktor, Surfactant-Protein C, Rhodopsin, γ-Kristallinen, Laktoferrin, βig-h3, Kerato-Epithelin, Corneodesmosin, p53, Lactadherin und Prolaktin.

7. Verfahren nach einem der Ansprüche 1 bis 6, wobei mindestens einer des einen Reportergens oder der mehreren Reportergene für ein Protein mit einer Aktivität codiert, die Wachstum und/oder Vermehrung der manipulierten Hefezellen positiv oder negativ beeinflusst.

8. Hefezelle mit einem Transgen, das als ein extrachromosomales Element oder integriert in seine genomische DNA vorhanden ist, wobei die Zelle umfasst:
a) ein oder mehrere Reportergen(e) unter transkriptionaler Kontrolle eines Promotors eines Hitzeschockproteins; und
b) ein oder mehrere Transgen(e), das oder die für ein oder mehrere Protein(e) codiert bzw. codieren, das oder die zur Fehlfaltung neigt bzw. neigen, wobei das Protein, das zur Fehlfaltung neigt, ausgewählt ist aus der Gruppe bestehend aus Serpinen, Poly(A)-Bindungsprotein 2, LDL-Rezeptor, Apolipoprotein B100, Cochlin, RET, Myelinprotein 22/0, kurzkettiger Acyl-CoA-Dehydrogenase, CFTR (Cystic Fibrosis Transmembrane Conductance Regulator), HERG, Phenylalaninhydroxylase, Fibrillin, Prokollagen, Kollagen, Hämoglobin, ATPase ATP7B, β-Hexosaminidase-Tyrosinase, amyloidem Vorläuferprotein, tau, α-Synuklein, Huntingtin, ADan Peptid, ABri Peptid, amyloidem β, Prionprotein, Ataxinen, Superoxiddesmutase, Cystatin C, Atrophin 1, Androgenrezeptor, TATA-Box-Bindungsprotein, Transthyretin, Serum-Amyloid A, Ig Leichtketten, β2-Mikroglobulin, Apolipoprotein A-1, Gelsolin, Pro-Islet-Amyloid-Polypeptid, Prokalzitonin, Lysozym, Insulin, Fibrinogen α-Kette, atrialem natriuretischen Faktor, Surfactant-Protein C, Rhodopsin, γ-Kristallinen, Laktoferrin, βig-h3, Kerato-Epithelin, Corneodesmosin, p53, Lactadherin und Prolaktin.

9. Zelle nach Anspruch 8, wobei mindestens eines des einen Proteins oder der mehreren Proteine, das oder die zu einer Fehlfaltung neigt bzw. neigen, ein amyloidogenes Protein ist, das ausgewählt ist aus der Gruppe bestehend aus amyloidem Vorläuferprotein, tau, α-Synuklein, Huntingtin, ADan Peptid, ABri Peptid, amyloidem β, Prionprotein, Ataxinen, Superoxiddesmutase, Cystatin C, Atrophin 1, Androgenrezeptor, TATA-Box-Bindungsprotein, Transthyretin, Serum-Amyloid A, Ig Leichtketten, β2-Mikroglobulin, Apolipoprotein A-1, Gelsolin, Pro-Islet-Amyloid-Polypeptid, Prokalzitonin, Lysozym, Insulin, Fibrinogen α-Kette, atrialem natriuretischen Faktor, Surfactant-Protein C, Rhodopsin, γ-Kristallinen, Laktoferrin, βig-h3, Kerato-Epithelin, Corneodesmosin, p53, Lactadherin und Prolaktin.

10. Kultur von Hefezellen nach Anspruch 8 oder 9, **dadurch gekennzeichnet, dass** die Zellen einen Expressionsvektor umfassen, der eine cDNA umfasst.

11. Verfahren zum Erzeugen einer Hefezelle zum Überwachen einer Proteinfehlfaltung, umfassend die folgenden Schritte:
a) Bereitstellen manipulierter Hefezellen, umfassend:
- ein oder mehrere Reportergen(e) unter transkriptionaler Kontrolle eines Promotors eines Hitzeschockproteins und
- ein oder mehrere Gen(e), die für ein oder mehrere Protein(e) codieren, die oder die zur Fehlfaltung neigt bzw. neigen,
b) Kultivieren der Hefezellen unter Bedingungen, die Mutationen des Genoms der Hefezellen ermöglichen; und
c) Selektieren einer Zelle, bei welcher ein oder mehrere Reportergen(e) bei höheren Werten im Vergleich zu Hefezellen vor der Kultivierung exprimiert werden, wobei eine Neigung zur Fehlfaltung besteht, wobei das Protein, das zu Fehlfaltung neigt, ausgewählt ist aus der Gruppe bestehend aus Serpinen, Poly(A)-Bindungsprotein 2, LDL-Rezeptor, Apolipoprotein B100, Cochlin, RET, Myelinprotein 22/0, kurzkettiger Acyl-CoA-Dehydrogenase, CFTR (Cystic Fibrosis Transmembrane Conductance Regulator), HERG, Phenylalaninhydroxylase, Fibrillin, Prokollagen, Kollagen, Hämoglobin, ATPase ATP7B, β-Hexosaminidase-Tyrosinase, amyloidem Vorläuferprotein, tau, α-Synuklein, Huntingtin, ADan Peptid, ABri Peptid, amyloidem β, Prionprotein, Ataxinen, Superoxiddesmutase, Cystatin C, Atrophin 1, Androgenrezeptor, TATA-Box-Bindungsprotein, Transthyretin, Serum-Amyloid A, lg Leichtketten, β2-Mikroglobulin, Apolipoprotein A-1, Gelsolin, Pro-Islet-Amyloid-Polypeptid, Prokalzitonin, Lysozym, Insulin, Fibrinogen α-Kette, atrialem natriuretischen Faktor, Surfactant-Protein C, Rhodopsin, γ-Kristallinen, Laktoferrin, βig-h3, Kerato-Epithelin, Corneodesmosin, p53, Lactadherin und Prolaktin.

12. Verfahren nach Anspruch 11, wobei die Zellen mit einem Gen versehen sind, das für einen Gegenselektionsmarker codiert, der an das eine oder die mehreren Gen(e) gebunden ist, das oder die für ein oder mehrere Protein(e) codiert bzw. codieren, das oder die zur Fehlfaltung neigt bzw. neigen, und das Verfahren den Schritt des Auswertens durch Verwendung des Gegenselektionsmarkers umfasst, ob die Reportergen-Expressionswerte von dem Vorhandensein des einen Gens oder der mehreren Gene abhängig sind, das oder die für ein oder mehrere Protein(e) codiert bzw. codieren, das oder die zur Fehlfaltung neigt bzw. neigen.

13. Verwendung einer Zelle nach Anspruch 8 oder 9 für die Identifizierung von Verbindungen und/oder Bedingungen, die die Proteinfehlfaltung beeinflussen, durch Nachweisen der Aktivität des Reportergenprodukts in den Zellen bei Anwendung der Verbindungen und/oder Bedingungen.

## Revendications

1. Procédé pour déterminer si un composé ou une pluralité de composés est/sont susceptibles de directement ou indirectement affecter un mauvais repliement protéique, ledit procédé comprenant les étapes de :
a) fourniture d'une culture de cellules de levure manipulées, comprenant un ou plusieurs gène(s) rapporteur(s) sous la commande transcriptionnelle d'un promoteur d'une protéine de choc thermique, et comprenant en outre un ou plusieurs gène(s) codant une ou plusieurs protéine(s) encline(s) à un mauvais repliement, dans lequel les cellules de levure manipulées sont **caractérisées par** un mauvais repliement spontané ou inductible de ladite ou desdites protéine(s) encline(s) à un mauvais repliement ; et
b) mise en contact des cellules avec ledit composé ou ladite pluralité de composés et détection de l'activité dudit produit formant gène rapporteur dans lesdites cellules pour déterminer l'effet dudit composé ou de ladite pluralité de composés sur un mauvais repliement protéique dans lesdites cellules manipulées.
dans lequel la protéine encline à un mauvais repliement est sélectionnée à partir du groupe constitué par des serpines, la protéine 2 de liaison au poly(A), le récepteur de lipoprotéine à basse densité, l'apolipoprotéine B100, la cochline, RET, la protéine de myéline 22/0, l'acyle CoA déshydrogénase à chaîne courte, le régulateur de conductance transmembranaire de la fibrose kystique, HERG, l'hydroxylase de phénylalanine, la fibrilline, le procollagène, le collagène, l'hémoglobine, l'ATPase ATP7B, la β-hexosaminidase tyrosinase, la protéine de précurseur amyloïde, tau, l'α-synucléine, l'huntingtine, le peptide ADan, le peptide ABri, l'amyloïde β, la protéine de prion, des ataxines, la superoxyde dismutase, la cystatine C, l'atrophine 1, le récepteur androgène, la protéine de liaison à la boîte TATA, la transthyrétine, le sérum amyloïde A, des chaînes légères Ig, la β2-microglobuline, l'apolipoprotéine A-1, la gelsoline, le pro-polypeptide de l'amyloïde des îlots, la procalcitonine, le lysozyme, l'insuline, une α-chaîne fibrinogène, un facteur natriurétique atrial, la protéine d'agent de surface C, la rhodopsine, des γ-cristallines, la lactoferrine, le βig-h3, la kérato-épithéline, la cornéodesmosine, p53, la lactadhérine et la prolactine.

2. Procédé selon la revendication 1, qui est un procédé pour cribler une pluralité de composés susceptibles de directement ou indirectement affecter un mauvais repliement protéique,
dans lequel lesdits composés sont des ADNc ou les produits d'expression de ceux-ci, comprenant les étapes de :
a) fourniture d'une culture de cellules de levure manipulées comprenant un ou plusieurs gène(s) rapporteur(s) sous la commande transcriptionnelle d'un promoteur d'une protéine de choc thermique, et un ou plusieurs gène(s) codant une ou plusieurs protéine(s) encline(s) à un mauvais repliement, la cellule de levure manipulée étant **caractérisée par** un mauvais repliement spontané ou inductible de ladite ou desdites protéine(s) encline(s) à un mauvais repliement ;
b) transformation de la culture de cellules manipulées avec une bibliothèque d'ADNc d'expression de façon à obtenir une culture de cellules transformées, chacune comprenant un ou plusieurs ADNc ; et,
c) détection de l'activité du gène rapporteur dans les cellules transformées de la culture pour déterminer l'effet du produit d'expression desdits un ou plusieurs ADNc sur un mauvais repliement dans lesdites cellules transformées.

3. Procédé selon la revendication 1 ou 2, dans lequel lesdites cellules de levure manipulées sont **caractérisées par** un mauvais repliement spontané de la ou des plusieurs protéine(s) encline(s) à un mauvais repliement et l'étape (a) comprend :
- la culture de cellules de levure manipulées comprenant un ou plusieurs gène(s) rapporteur(s) sous la commande transcriptionnelle d'un promoteur d'une protéine de choc thermique et un ou plusieurs gène(s) codant une ou plusieurs protéine(s) encline(s) à un mauvais repliement, sous des conditions qui permettent et/ou induisent des mutations du génome des cellules de levure manipulées aboutissant à un mauvais repliement spontané de la ou des protéine(s) encline(s) à un mauvais repliement, et
- la sélection d'une cellule **caractérisée par** un mauvais repliement spontané basé sur l'expression accrue du ou des plusieurs gène(s) rapporteur(s) en comparaison aux cellules de levure manipulées avant culture.

4. Procédé selon l'une quelconque des revendications 1 à 3, dans lequel lesdites cellules sont **caractérisées par** un mauvais repliement inductible de la ou des protéine(s) encline(s) à un mauvais repliement et le procédé comprend en outre, avant l'étape (b) l'étape de mise en contact des cellules de levure manipulées avec un ou plusieurs facteurs externes susceptibles d'induire de manière sélective un mauvais repliement de la ou des plusieurs protéines enclines à un mauvais repliement.

5. Procédé selon la revendication 4, dans lequel au moins une de ladite ou desdites protéine(s) encline(s) à un mauvais repliement est une α-synucléine et le facteur externe susceptible d'induire de manière sélective un mauvais repliement de la ou des plusieurs protéine(s) encline(s) à mauvais repliement est sélectionné à partir du groupe constitué par le paraquat, la roténone et MPTP.

6. Procédé selon l'une quelconque des revendications 1 à 5, dans lequel au moins une de ladite ou desdites plusieurs protéine(s) encline(s) à un mauvais repliement protéique est une protéine amyloïdogénique sélectionnée à partir du groupe constitué par la protéine de précurseur amyloïde, tau, l'α-synucléine, l'huntingtine, le peptide ADan, le peptide ABri, le β-amyloïde, la protéine de prion, des ataxines, la superoxyde dismutase, la cystatine C, l'atrophine 1, le récepteur androgène, la protéine de liaison à la boîte TATA, la transthyrétine, le sérum amyloïde A, des chaînes légères Ig, la β2-microglobuline, l'apolipoprotéine A-1, la gelsoline, le pro-polypeptide de l'amyloïde des îlots, la procalcitonine, le lysozyme, l'insuline, une α-chaîne fibrinogène, un facteur natriurétique atrial, la protéine d'agent de surface C, la rhodopsine, des γ-cristallines, la lactoferrine, le βig-h3, la kérato-épithéline, la cornéodesmosine, p53, la lactadhérine et la prolactine.

7. Procédé selon l'une quelconque des revendications 1 à 6, dans lequel au moins un desdits un ou plusieurs gène(s) rapporteur(s) code une protéine avec une activité qui affecte positivement ou négativement la croissance et/ou la prolifération desdites cellules de levure manipulées.

8. Cellule de levure, ayant un transgène présent en tant qu'élément extrachromosomique ou intégré dans son ADN génomique, ladite cellule comprenant :
a) un ou plusieurs gène (s) rapporteur(s) sous la commande transcriptionnelle d'un promoteur d'une protéine de choc thermique ; et
b) un ou plusieurs transgène(s) codant une ou plusieurs protéine(s) encline(s) à un mauvais repliement, dans lequel la protéine encline à un mauvais repliement est sélectionnée à partir du groupe constitué par des serpines, la protéine 2 de liaison au poly(A), le récepteur de lipoprotéine à basse densité, l'apolipoprotéine B100, la cochline, RET, la protéine de myéline 22/0, l'acyle CoA déshydrogénase à chaîne courte, le régulateur de conductance transmembranaire de la fibrose kystique, HERG, l'hydroxylase de phénylalanine, la fibrilline, le procollagène, le collagène, l'hémoglobine, l'ATPase ATP7B, la β-hexosaminidase tyrosinase, la protéine de précurseur amyloïde, tau, l'α-synucléine, l'huntingtine, le peptide ADan, le peptide ABri, l'amyloïde β, la protéine de prion, des ataxines, la superoxyde dismutase, la cystatine C, l'atrophine 1, le récepteur androgène, la protéine de liaison à la boîte TATA, la transthyrétine, le sérum amyloïde A, des chaînes légères Ig, la β2-microglobuline, l'apolipoprotéine A-1, la gelsoline, le pro-polypeptide de l'amyloïde des îlots, la procalcitonine, le lysozyme, l'insuline, une α-chaîne fibrinogène, un facteur natriurétique atrial, la protéine d'agent de surface C, la rhodopsine, des γ-cristallines, la lactoferrine, le βig-h3, la kérato-épithéline, la cornéodesmosine, p53, la lactadhérine et la prolactine.

9. Cellule selon la revendication 8, dans laquelle au moins une desdites une ou plusieurs protéines enclines à un mauvais repliement est une protéine amyloïdogénique sélectionnée à partir du groupe constitué par la protéine de précurseur amyloïde, tau, l'α-synucléine, l'huntingtine, le peptide ADan, le peptide ABri, le β-amyloïde, la protéine de prion, des ataxines, la superoxyde dismutase, la cystatine C, l'atrophine 1, le récepteur androgène, la protéine de liaison à la boîte TATA, la transthyrétine, le sérum amyloïde A, des chaînes légères Ig, la β2-microglobuline, l'apolipoprotéine A-1, la gelsoline, le pro-polypeptide de l'amyloïde des îlots, la procalcitonine, le lysozyme, l'insuline, une α-chaîne fibrinogène, un facteur natriurétique atrial, la protéine d'agent de surface C, la rhodopsine, des γ-cristallines, la lactoferrine, le βig-h3, la kérato-épithéline, la cornéodesmosine, p53, la lactadhérine et la prolactine.

10. Culture de cellules de levure selon la revendication 8 ou 9, **caractérisée en ce que** lesdites cellules comprennent un vecteur d'expression comprenant un ADNc.

11. Procédé pour produire une cellule de levure pour contrôler un mauvais repliement protéique, comprenant les étapes de :
a) fourniture de cellules de levure manipulées comprenant :
- un ou plusieurs gène(s) rapporteur(s) sous la commande transcriptionnelle d'un promoteur d'une protéine de choc thermique, et
- un ou plusieurs gènes codant une ou plusieurs protéines enclines à un mauvais repliement,
b) culture desdites cellules de levure sous des conditions qui permettent des mutations du génome desdites cellules de levure ; et
c) sélection d'une cellule dans laquelle lesdits un ou plusieurs gène(s) rapporteur(s) sont exprimés à des niveaux supérieurs, en comparaison aux cellules de levure avant la culture
dans lequel la protéine encline à un mauvais repliement est sélectionnée à partir du groupe constitué par des serpines, la protéine 2 de liaison au poly(A), le récepteur de lipoprotéine à basse densité, l'apolipoprotéine B100, la cochline, RET, la protéine de myéline 22/0, l'acyle CoA déshydrogénase à chaîne courte, le régulateur de conductance transmembranaire de la fibrose kystique, HERG, l'hydroxylase de phénylalanine, la fibrilline, le procollagène, le collagène, l'hémoglobine, l'ATPase ATP7B, la β-hexosaminidase tyrosinase, la protéine de précurseur amyloïde, tau, l'α-synucléine, l'huntingtine, le peptide ADan, le peptide ABri, l'amyloïde β, la protéine de prion, des ataxines, la superoxyde dismutase, la cystatine C, l'atrophine 1, le récepteur androgène, la protéine de liaison à la boîte TATA, la transthyrétine, le sérum amyloïde A, des chaînes légères Ig, la β2-microglobuline, l'apolipoprotéine A-1, la gelsoline, le pro-polypeptide de l'amyloïde des îlots, la procalcitonine, le lysozyme, l'insuline, une α-chaîne fibrinogène, un facteur natriurétique atrial, la protéine d'agent de surface C, la rhodopsine, des γ-cristallines, la lactoferrine, le βig-h3, la kérato-épithéline, la cornéodesmosine, p53, la lactadhérine et la prolactine.

12. Procédé selon la revendication 11, dans lequel les cellules sont munies d'un gène codant un marqueur de contre-sélection lié auxdits un ou plusieurs gènes codant une ou plusieurs protéines enclines à un mauvais repliement et ledit procédé comprend l'étape d'évaluation, par l'utilisation dudit marqueur de contre-sélection, si les niveaux d'expression de gène rapporteur dépendent de la présence desdits un ou plusieurs gènes codant une ou plusieurs protéines enclines à un mauvais repliement.

13. Utilisation d'une cellule selon la revendication 8 ou 9, pour l'identification de composés et/ou de conditions affectant un mauvais repliement protéique, en détectant l'activité dudit produit formant gène rapporteur dans lesdites cellules lors d'une application desdits composés et/ou desdites conditions.
